(19) Europäisches Patentamt
European Patent Office
Office européen des brevets

(11) EP 3 388 518 A1

(12) EUROPEAN PATENT APPLICATION

(43) Date of publication:
**17.10.2018 Bulletin 2018/42**

(51) Int Cl.:
*C12N 9/88* (2006.01) *C12P 7/26* (2006.01)
*C12P 7/38* (2006.01) *C12P 7/24* (2006.01)

(21) Application number: **17382194.3**

(22) Date of filing: **10.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Consejo Superior de Investigaciones Científicas (CSIC)**
  **28006 Madrid (ES)**
- **Technical University of Darmstadt**
  **64287 Darmstadt (DE)**
- **Sustainable Momentum, S.L.**
  **35003 Las Palmas de gran Canaria (ES)**

(72) Inventors:
- **CLAPÉS SABORIT, Pedro**
  **08034 Barcelona (ES)**
- **JOGLAR TAMARGO, Jesús**
  **08034 Barcelona (ES)**
- **BUJONS VILÁS, Jorge**
  **08034 Barcelona (ES)**
- **ROLDÁN GARCÍA, Raquel**
  **08034 Barcelona (ES)**
- **FESSNER, Wolf-Dieter**
  **64287 Darmstadt (DE)**
- **JUNKER, Sebastian**
  **64287 Darmstadt (DE)**
- **DOMINGUEZ DE MARÍA, Pablo**
  **35004 Las Palmas de Gran Canaria (ES)**

(74) Representative: **Pons Ariño, Angel**
**Pons Patentes y Marcas Internacional, S.L.**
**Glorieta Rubén Dario 4**
**28010 Madrid (ES)**

(54) **FRUCTOSE-6-PHOSPHATE ALDOLASE VARIANTS FOR ALDOL CARBOLIGATIONS**

(57) The invention provides new and alternative fructose-6-phosphate aldolase (FSA) variants which enable the production of optically active building blocks with high chemoselectivity and stereoselectivity using aldehydes as starting material in aldol carboligation reactions, while avoiding the by-product formation and subsequent reactions.

EP 3 388 518 A1

SEQUENCE LISTING

<110> Consejo Superior de Investigaciones Cient\355ficas (CSIC)
Technical University of Darmstadt
Sustainable Momentum S. L.

<120> FRUCTOSE-6-PHOSPHATE ALDOLASE VARIANTS FOR ALDOL CARBOLIGATIONS

<130> EP1641.1281

<160> 1

<170> PatentIn version 3.5

<210> 1
<211> 220
<212> PRT
<213> Escherichia coli

<220>
<221> MUTAGEN
<222> (6)..(6)
<223> Amino acid at this position may be substituted by any of the amino acids A, L, N, Q, S, T, E, H, V, G, I or P

<220>
<221> MUTAGEN
<222> (26)..(26)
<223> Amino acid at this position may be substituted by any of the amino acids V, A, L, I or P

<220>
<221> MUTAGEN
<222> (165)..(165)
<223> Amino acid at this position may be substituted by the amino acid G

<400> 1

```
Met Glu Leu Tyr Leu Asp Thr Ser Asp Val Val Ala Val Lys Ala Leu
1               5                   10                  15

Ser Arg Ile Phe Pro Leu Ala Gly Val Thr Thr Asn Pro Ser Ile Ile
            20                  25                  30

Ala Ala Gly Lys Lys Pro Leu Asp Val Val Leu Pro Gln Leu His Glu
        35                  40                  45

Ala Met Gly Gly Gln Gly Arg Leu Phe Ala Gln Val Met Ala Thr Thr
    50                  55                  60

Ala Glu Gly Met Val Asn Asp Ala Leu Lys Leu Arg Ser Ile Ile Ala
65                  70                  75                  80

Asp Ile Val Val Lys Val Pro Val Thr Ala Glu Gly Leu Ala Ala Ile
                85                  90                  95
```

```
Lys Met Leu Lys Ala Glu Gly Ile Pro Thr Leu Gly Thr Ala Val Tyr
            100                 105                 110

Gly Ala Ala Gln Gly Leu Leu Ser Ala Leu Ala Gly Ala Glu Tyr Val
        115                 120                 125

Ala Pro Tyr Val Asn Arg Ile Asp Ala Gln Gly Gly Ser Gly Ile Gln
    130                 135                 140

Thr Val Thr Asp Leu His Gln Leu Leu Lys Met His Ala Pro Gln Ala
145                 150                 155                 160

Lys Val Leu Ala Ala Ser Phe Lys Thr Pro Arg Gln Ala Leu Asp Cys
                165                 170                 175

Leu Leu Ala Gly Cys Glu Ser Ile Thr Leu Pro Leu Asp Val Ala Gln
            180                 185                 190

Gln Met Ile Ser Tyr Pro Ala Val Asp Ala Ala Val Ala Lys Phe Glu
        195                 200                 205

Gln Asp Trp Gln Gly Ala Phe Gly Arg Thr Ser Ile
    210                 215                 220
```

## Description

The invention relates to the field of chemistry, specifically to aldol carboligation reactions. More particularly, the invention refers to aldolase enzyme variants that can be used as improved biocatalysts in these reactions, because they selectively produce optically active compounds with high stereo- and chemoselectivity, avoiding by-product formation.

## BACKGROUND ART

The aldol reaction is a whole class of reactions between enolates and carbonyl compounds. These are very important reactions in organic synthesis, because when the nucleophilic enolate attacks the electrophilic aldehyde or ketone, a new carbon-carbon bond is formed. Among different types of aldol reactions, aldehydes are of particular interest as donor molecules, because the product formed are aldehydes themselves and they can be subjected to further addition and lead to more complex building blocks.

The enantioselective aldol carboligation of aldehydes to afford optically active compounds is an attractive strategy to generate value. Starting materials are often readily available and inexpensive, and the obtained products are very versatile for many chemical segments and have high added value. However, despite the potential that such reactions may have, their practical use is hampered in many cases due to the intrinsic high reactivity of aldehydes, what makes these reactions prone to suffer from by-product formation, crotonizations, oligomerizations, racemization and other subsequent (and parallel) reactions. The needed purification of the desired target products from the (aqueous) complex crude mixture of by-products obtained at the end of the reaction is rather cumbersome, and in many cases virtually impossible from a practical viewpoint. Thus, these synthetic approaches cannot be used at industrial level.

An illustrative example of this problem would be the cross carboligation between chloro-ethanal and ethanal to (selectively) afford 4-chloro-3-hydroxy-butanal (3):

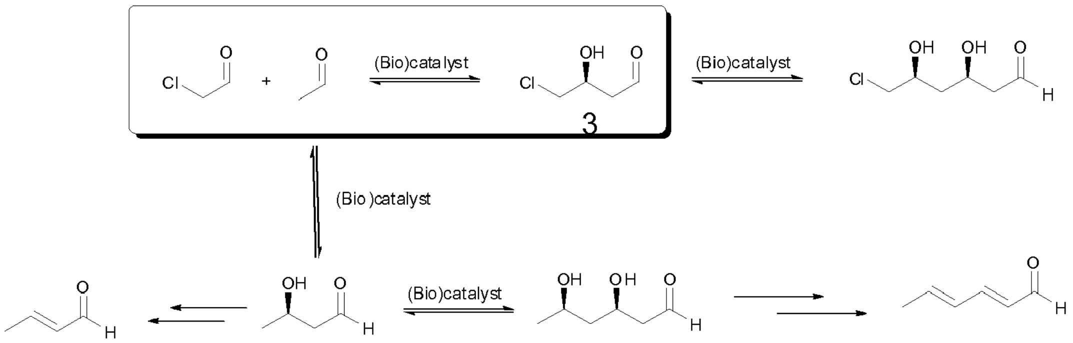

3

This reaction represents an example of the challenging selective cross carboligation between highly reactive aldehydes. In the square the desired (selective) process is depicted, whereas in the rest of the figure some of the possible by-products that are typically observed in crude mixtures of those reactions are shown.

In this exemplary case, compound (3) may be a useful building block for pharmaceutical and fine chemical applications, as well as for natural compounds synthesis. Yet the process is hampered by the number of by-products and subsequent (and parallel) reactions that may take place, either catalyzed or spontaneously produced. An example of this complexity and kinetics of the mixed processes can be found on literature as well (Rucigaj and Krajnc, Chem. Eng. J. 2015, 259, 11-24).

Given the importance that, for instance, 4-chloro-3-hydroxy-butanal (3) may have for synthetic purposes, several patents disclose its synthesis. Thus, CN103145540 reports on the ozonization of 5-halogenated-4-hydroxy-1-pentene compounds, leading to the desired aldehyde, which is further used *via* Wittig-type reactions and epoxidations, with application in the synthesis of natural products. Likewise, US8742176 discloses the use of pyrrolydine-based organocatalysis for the enantioselective addition of ethanal to chloroethanal, again with subsequent Wittig-type condensation and epoxidation. With regard to biocatalysis, EP1734129 focuses on the enzyme 2-deoxyribose-5-phosphate aldolase (DERA) for the production of (3), as well as on the further addition of another ethanal molecule to (3), to afford the statin-chain precursor. In that patent, it is mentioned that DERA from *Pyrobaculum aerophilum* is selective for the mono-addition of two aldehydes (namely choroethanal and ethanal, among other examples). However, a careful reading of that patent shows that such enzyme displays not-complete selectivity for the single addition, but the double addition is still present (example 7, page 12 of said patent). Thus, a chromatographic separation of the single and the double addition adducts is still needed at the end of the reaction.

Aldol reactions have been extensively studied from different perspectives including the development of new catalysts for stereocontrolled synthesis. Catalysts that have been developed for asymmetric aldol reactions include small organic molecules, transition metal coordination complexes with chiral ligands and aldolases. The synthetic utility of aldol reactions would be greatly enhanced if the formation of enolate anion and the attack of carbonyl group could be chemically selective in a cross aldol reaction to avoid the formation of complex products mixture. Since enzymes exert high level of selectivity that is not usually possible in simple chemical reaction in the laboratory, aldolases have emerged as an environmentally friendly alternative with great application potentials in the synthesis of rare sugars or sugar derivatives such as statins, iminocyclitols, epothilones and sialic acids.

Aldolases (EC 4.1.2.X) are lyases that typically catalyze the stereoselective addition of a ketone or aldehyde donor to an aldehyde acceptor. Up until now, there are more than 30 known aldolases present in most, if not all, organisms. Aldolases are nature's own catalysts for one of the most fundamental reactions in organic chemistry: the formation of new carbon-carbon bonds. In biological systems, aldol formation and cleavage reactions play central roles in sugar metabolism. In organic synthesis, as previously mentioned, aldolases attract great attention as environmentally friendly alternative for the synthesis of polyhydroxylated compounds in stereocontrolled manner.

Thus, approaches for designing aldolases with improved donor selectivity have been developed. For instance, fructose-6-phosphate aldolase (FSA) variants from *E. coli* with single, double or triple amino acid substitutions have been described in Anna Szekrenyi, et al., 2014, Chemistry A European Journal, Engineering the Donor Selectivity of D-Fructose-6-Phosphate Aldolase for Biocatalytic Asymmetric Cross-Aldol Additions of Glycolaldehyde. Specifically in this prior art document, the following *E. coli* FSA variants were disclosed: L107Y, A129G/A165G, A129S/A165G, A129T/A165G, A129G, A129V, A129T, A129S, L107Y/A129G, L107H/A129G, L107F/A129G, L107H, L107Y/A129G/A165G, L107H/A129G/A165G and L107F. The selectivity of these variants towards donor substrates, such as glycolaldehyde and dihydroxyacetone, was assessed. Other FSA variants, such as A165G, A129S and A165G/A129S have been found to be selective for additions of hydroxypropanone and dihydroxypropanone (Gutierrez, et al., Chem. Commun. 2011, 47 (20), 5762-5764). However, whereas all these variants are selective for hydroxymethyl ketones as nucleophile components they are inactive for those without the hydroxymethyl functionality. Therefore, no FSA variants have been described to be active toward nucleophiles without hydroxymethyl functionality and non-phosphorylated electrophiles.

Furthermore, *E. coli* FSA variants with increased catalytic activity for aldol reaction with phenylacetaldehyde and hydroxyacetone as substrates have been described in Huan Ma. 2015. Digital Comprehensive Summaries of Uppsala Dissertations from the Faculty of Science and Technology 1318. 67. Particularly, these variants were obtained by mutagenesis around the substrate binding site near catalytic Lys85, Arg134 and Ser166. However, the same drawbacks as those indicated above for the FSA variants of the state of the art can be applied for these variants.

As can be seen, based on the synthetic potential of compounds such as 4-chloro-3-hydroxy-butanal, (3), and others - as well as their expected high market prices -, there is a need for selective (bio)catalysts that may synthetize exclusively the desired target, while avoiding the by-product formation and subsequent reactions, as well as further downstream processing units. Having those (bio)catalysts in hands, the production of highly valuable (optically active) building blocks with high chemoselectivity and stereoselectivity would facilitate the set-up of economical approaches using lyases (aldolases) and highly reactive (inexpensive) aldehydes as starting materials.

To date, no such high stereoselective-(bio)catalysts exist in the literature for the intended reactions, and only DERA from *Pyrobaculum aerophilum* seems to show some selectivity for (3) compared to subsequent additions of aldehydes to the formed product (3). Therefore, it is industrially very important to develop (bio)catalysts for producing predominantly the desired product, with high chemo- and stereoselectivity, in aldol carboligation reactions from aldehydes.

## DESCRIPTION OF THE INVENTION

This invention is related to enzymatic processes based on *E. coli* fructose-6-phosphate aldolase (FSA) variants catalysis which provides chiral synthons and other products with high potential interest for synthetic processes with excellent chemoselectivity and stereoselectivity under environmentally friendly conditions.

Particularly, the invention provides new and alternative FSA variants (mutants) which enable the production of highly valuable (optically active) building blocks with high chemoselectivity and stereoselectivity using aldehydes as starting material in aldol carboligation reactions, while avoiding the by-product formation and subsequent reactions.

The present invention discloses several FSA variants that are suitable and advantageous as enzymatic biocatalysts for the chemoselective single C-C bond formation of different building blocks. When using these FSA variants of the invention, the by-products expected in these aldol carboligation reactions (see figure above) are not found, since these variants are fully selective for the single addition of the aldehydes used as substrates. Therefore, FSA variants of the invention are highly selective and only two substrates are reacting in their presence to produce a single condensed product, the reaction stops at that desired compound, i. e. only the desired product is formed. This approach allows thus the use of highly reactive aldehydes as substrates.

The invention provides, therefore, an enantioselective or diastereoselective biocatalytic aldol reaction (C-C bond formation) using aldehydes and ketones as substrates, leading exclusively to the single, chemo- and stereoselective carboligation of two substrates - which may be similar or different between them - with no formation of by-products or further consecutive reactions adding more aldehydes. Selectivity in the single addition is always > 99.99 %, as the FSA aldolase variants of the invention, when used as biocatalysts, show exclusive selectivity for the single aldol addition of two substrates.

Therefore, the designed FSA variants of the invention have remarkable high selectivity in so challenging reactions, producing compounds with high potential interest for synthetic processes, whereas the FSA wild-types are not able to catalyze these reactions at all.

No other (bio)catalysts able to perform these reactions are known, being so selective, in which only two substrates are reacting to produce a single condensed product and reaction stops at that desired compound. The only example is the above-reported DERA, in which some (incomplete) selectivity for the single addition is noted. In the present invention, as a remarkable example for the FSA variants, in the case of compound 4-chloro-3-hydroxy-butanal (3), very promising productivities (space-time-yields of 10-12 g $L^{-1}d^{-1}$) have been achieved. This suggests that after careful fine-tuning and optimization, robust and fully selective biocatalysts have been obtained in the present invention. Moreover, these aldol carboligation reactions have been studied in the presence of the *E. coli* FSA wild type or each of the following FSA variants: A165G, L107A, L163A, A129G, L107Y/A129G, A129V, A129T, A129S, L107Y/A129G, L107H/A129G, L107F/A129G, L107H, L107Y/A129G/A165G, L107H/A129G/A165G, L107F, L107Y/A129G/A165G/S166G, but reactions did not take place or proceeded with low chemo- and stereoselectivity.

Moreover, these FSA variants disclosed in the present invention are selective for both hydroxymethyl ketones as nucleophile components and for those without the hydroxymethyl functionality. No FSA variants had been described up to date to be active toward nucleophiles without hydroxymethyl funcionality and non-phosphorylated electrophiles.

As explained above, aldol reactions are very useful but when highly reactive aldehydes are employed their practical use is hampered. Work-up of a complex mixture is virtually uneconomic. However, with the new FSA-variants-based approach presented herein downstream is significantly simplified, as only the desired product is formed. Moreover, given the mild conditions of the processes, it is possible to perform enzymatic multi-step processes to further derivatization of the obtained compounds.

Thus, the present invention solves the aforementioned problems by providing FSA aldolase variants that selectively produce the desired product as well as a process for producing optically active compounds with high chemo- and stereoselectivity.

Specifically, the inventors found that mutagenesis (preferably, amino acid substitutions) at a particular position, namely position 6, of the SEQ ID NO: 1 (which is the wild type amino acid sequence of the FSA from *Escherichia coli*) gives FSA variants that lead to a high chemo- and stereoselectivity when used as biocatalysts in aldol carboligation reactions. This results in FSA variants producing predominantly the desired optically active product with high selectivity, preferably with improved selectivity compared to the wild type sequence of SEQ ID NO: 1. Therefore, the inventors propose this position 6 in the amino acid sequence of the wild type FSA enzyme from *E. coli* for the design of FSA enzyme variants that lead to a high chemo- and stereoselectivity in aldol carboligation reactions. This particular amino acid residue located at position 6 of the FSA sequence SEQ ID NO: 1 has not been associated before to the substrate specificity or the stereoselectivity of the enzyme.

Thus, a first aspect of the invention refers to a fructose-6-phosphate aldolase variant comprising:

a. an amino acid sequence having at least 80%, preferably at least 85%, 90%, 91%, 92%, 93%, 94%, 95%, 96%, 97%, 98% or 99%, more preferably at least 90%, more preferably at least 95%, even more preferably at least 99%, sequence identity with SEQ ID NO: 1, and

b. an amino acid substitution at position 6, corresponding to positions 1 to 220 of SEQ ID NO: 1.

Hereinafter, this variant will be also referred to as "variant of the invention" or "FSA variant of the invention."

The FSA variants encompassed within the present invention lead to a high enantioselectivity or diastereoselectivity when used as a biocatalyst in a reaction of aldol carboligation, more preferably to an enhanced, increased or improved enantioselectivity or diastereoselectivity compared to the FSA of SEQ ID NO: 1.

The term "enantioselectivity" means the selectivity of a reaction towards one of a pair of enantiomers. The property of "enantioselectivity" may be assessed by any of the methods known in the art for the determination of enantioselectivity. For instance, the enantioselectivity may be determined by measuring the enantiomeric excess after the aldol carboligation reaction performed in the presence of the FSA variant of the invention. The term "enantiomeric excess" (ee) refers to the difference between the amounts of each of the enantiomers present in a mixture, relative to the total amount of the compound in the mixture expressed as percentage (x 100%). For an excess of the R-enantiomer, the enantiomeric excess can be calculated by the following formula:

$$ee\ \% = ([R] - [S]) / ([R] + [S]) \times 100$$

For an excess of the S-enantiomer, the enantiomeric excess can be calculated by the following formula:

$$ee\ \% = ([S] - [R]) / ([R] + [S]) \times 100$$

In the above formulas, [R] and [S] are the respective molar fractions of the enantiomers in a mixture such that [R] + [S] = 1.
The amounts of each of the enantiomers present in a mixture may be measured, for instance but without limitation, by HPLC, Chiral GC, and the like.
The term "diastereoselectivity" means the selectivity of a reaction towards one of a pair of diastereoisomers. The property of "diastereoselectivity" may be assessed by any of the methods known in the art for the determination of diastereoselectivity. For instance, the diastereoselectivity may be determined by measuring the diastereomeric ratio after the aldol carboligation reaction performed in the presence of the FSA variant of the invention. The term "diastereomeric ratio" (dr) refers to the ratio of the percentage of one diastereoisomer in a mixture to that of the other.
The amounts of each of the diastereoisomers present in a mixture may be measured, for instance but without limitation, by NMR.
According to the present invention, the enantioselectivity or diastereoselectivity of a reaction in which the FSA variant of the invention is used as a biocatalyst is "high" when the enantiomeric excess is, preferably, 95% or higher, more preferably 99% or higher, or the diastereomeric ratio is, preferably, 95 or higher, more preferably 99 or higher.
"Fructose-6-phosphate aldolase" or "FSA" is the enzyme classified as EC 4.1.2.X and the most recently discovered member of the Class I aldolase family. It exists as isoenzymes: FSAA and FSAB coded by *mipB* and *talC* genes in *E. coli*, respectively. The two isoenzymes have similar substrate profiles but FSAB shows lower catalytic activity and thermostability. FSA is the first reported enzyme capable of catalyzing the cleavage of fructose-6-phosphate to generate DHA and G3P. The amino acid sequence of wild type FSA referred to in the present invention is SEQ ID NO: 1.
The term "variant", as used herein, relates to a FSA enzyme that comprises at least one mutation, preferably at least one amino acid substitution, compared to the *E. coli* wild type FSA (SEQ ID NO: 1) at the points(s) described herein within its amino acid sequence and, therefore, has a different sequence to that of the natural or wild-type enzyme of SEQ ID NO: 1. As used herein, the expression "FSA variant" means preferably a polypeptide comprising the amino acid substitutions described herein and having fructose-6-phosphate aldolase activity, which have been produced by chemical synthesis or recombinantly by an organism that expresses a nucleotide sequence that encodes the FSA wild type, preferably of SEQ ID NO: 1, modified in the sense described herein. Said modified sequence is obtained by means of human intervention by modifying the nucleotide sequence that encodes the native or wild type FSA (preferably the wild type FSA of SEQ ID NO: 1). The term "modification" means any chemical modification of the amino acid or nucleic acid sequence of native or wild type FSA, preferably of SEQ ID NO: 1.
The term "identity" or "homology", as used herein, in the context of describing two or more polypeptide sequences, relates to a specified percentage of coincidences of amino acid residues at the positions from an alignment of two amino acid sequences. Sequence alignment methods for comparison are well known in the state of the art. The degree of identity can be determined using the Clustal method (Higgins, 1989, CABIOS 5: 151-153), the Wilbur-Lipman method (Wilbur y Lipman, 1983, Proceedings of the National Academy of Science USA 80: 726-730), the GAG program, including GAP (Devereux et al. 1984, Nucleic Acids Research 12: 287 Genetics Computer Group University of Wisconsin, Madison, 25 (WI)); BLAST or BLASTN, EMBOSS Needle and FASTA (Altschul et al. 1999, J. Mol. Biol. 215: 403-410). Additionally, the Smith-Waterman algorithm can also be used for the purpose of determining the degree of identity between two sequences. Preferably, the degree of identity is determined using BLAST with the default parameters. This software is publicly available at the *National Center for Biotechnology Information* (NCBI).
The FSA variants of the invention, apart from the amino acid substitutions at the specific positions indicated herein, can exhibit other limited changes in their amino acid sequences. These changes make it possible to maintain the fructose-6-phosphate aldolase activity of the FSA variants of the invention and maintain the property of leading to a high chemo- and stereoselectivity in aldol carboligation reactions. Said changes may be substitutions, deletions or additions. Substitutions are by conserved amino acids, which are amino acids with lateral strands and properties similar with respect to, for example, hydrophobic or aromatic properties. These substitutions include, but are not limited to, substitutions between Glu and Asp, Lys and Arg, Asn and Gln, Ser and Thr, and/or between the amino acids included in the following list: Ala, Leu, Val and Ile. The changes referred to in this paragraph do not lead to relevant modifications in the essential characteristics or properties of the variants of the invention.
The individual amino acids in an amino acid sequence are represented herein as XN, where X is the amino acid in the sequence (designated by means of the one letter code universally accepted in amino acid nomenclature)

and N is the position in the sequence. The amino acid substitutions are represented herein as $X_1NX_2$, where $X_1$ is the amino acid in the sequence of the non-mutated enzyme, $X_2$ is the new amino acid in the sequence of the mutated enzyme (variant) and N is the position in the amino acid sequence in relation to the positions of SEQ ID NO: 1.

In a preferred embodiment of the variant of the invention, the amino acid substitution at position 6 is by an amino acid selected from the list consisting of: A, L, N, Q, S, T, E, H, V, G, I or P, more preferably by H, N, Q, L, T, E or A. More preferably, the amino acid substitution is selected from the list consisting of: D6A, D6L, D6N, D6Q, D6S, D6T, D6E, D6H, D6V, D6G, D6I or D6P, preferably D6H, D6N, D6Q, D6L, D6T, D6E or D6A.

In a more preferred embodiment, the variant of the invention comprises, even more preferably consists of, the amino acid sequence SEQ ID NO: 1 in which the D at position 6 (D6) has been substituted by an amino acid selected from the list consisting of: A, L, N, Q, S, T, E, H, V, G, I or P, preferably by H, N, Q, L, T, E or A.

In an even more preferred embodiment, the variant of the invention comprises the amino acid substitution D6N or D6H. In a particular embodiment of the present invention, the variant comprises, even more preferably consists of, the amino acid sequence SEQ ID NO: 1 in which the D at position 6 (D6) has been substituted by N or H.

In another preferred embodiment, the variant of the invention further comprises an amino acid substitution at position 26, corresponding to positions 1 to 220 of SEQ ID NO: 1. More preferably, this amino acid substitution at position 26 is by an amino acid selected from the list consisting of: V, A, L, I or P, more preferably by L or I. Even more preferably, this amino acid substitution is selected from the list consisting of: T26V, T26A, T26L, T26I or T26P, preferably T26L or T26I.

In a particular embodiment, the variant of the invention comprises the amino acid substitutions D6A and T26L or D6A and T26I.

In another preferred embodiment, the variant of the invention further comprises an amino acid substitution at position 165, corresponding to positions 1 to 220 of SEQ ID NO: 1, by the amino acid G. More preferably, this amino acid substitution is A165G.

In another particular embodiment, the variant of the invention comprises the amino acid substitutions D6E and A165G or D6L and A165G.

In another particular embodiment, the variant of the invention comprises amino acid substitutions at positions 6, 26 and 165, corresponding to positions 1 to 220 of SEQ ID NO: 1, more preferably by any of the amino acids indicated above for each position.

Amino acid substitutions described herein introduced in the wild-type polypeptide sequence of FSA can be obtained using genetic engineering techniques or recombinant DNA, such as for example by mutating the encoding sequence of the wild type FSA enzyme by means of directed mutagenesis, or they can be obtained by means of chemical synthesis of the nucleotide sequence which codes for the sequence of the variant of the invention that carries said amino acid substitutions.

Therefore, the FSA variant of the invention can be synthesised, for example, but without limitations, *in vitro.* For example, by means of the synthesis of solid-phase peptides or recombinant DNA approaches. The variant of the invention can be produced in a recombinant manner, including its production as a mature peptide or as a pre-protein that includes a signal peptide.

Another aspect of the invention refers to an isolated nucleic acid sequence encoding the variant of the invention, hereinafter "nucleotide sequence of the invention".

Due to the degeneration of the genetic code, various nucleotide sequences can encode the same amino acid sequence.

In accordance with the present invention, an isolated "nucleic acid molecule", "nucleotide sequence", "nucleic acid sequence" or "polynucleotide" is a nucleic acid molecule (polynucleotide) that has been eliminated from its natural medium (*i.e.* it has been subjected to human manipulation) and can include DNA, RNA or DNA or RNA derivatives, including cDNA. The nucleotide sequence of the present invention may or may not be chemically or biochemically modified and can be artificially obtained by means of cloning and selection methods or by means of sequencing.

The nucleic acid sequence of the invention can encode the mature polypeptide or a pre-protein consisting of a signal peptide linked to the mature enzyme that must subsequently be processed.

The nucleotide sequence of the present invention may also comprise other elements, such as introns, non-encoding sequences at 3' and/or 5' ends, ribosome binding sites, restriction sites, etc. This nucleotide sequence can also include encoding sequences for additional amino acids that are useful for the purification or stability of the encoded peptide.

The nucleic acid sequence of the invention can be included in a genetic construct, preferably in an expression vector. Said genetic construct may also comprise one or more gene expression-regulating sequences, such as promoters, enhancers, regulators, terminators, etc.

Therefore, another aspect of the invention refers to a gene construct comprising the nucleic acid sequence of the invention, hereinafter "gene construct of the invention". In a preferred embodiment, said gene construct is an expression vector.

The expression "gene construct", "genetic construct" or "nucleic acid construct" as used herein relates to a

functional unit required to transfer, and preferably to express, a gene of interest, herein the nucleotide sequence of the invention as described, in a host cell. This gene construct preferably also comprises regulating or control sequences including, for example, a promoter, an enhancer, a terminator, etc. operably linked to the sequence that encodes the protein. It refers to a mono or double-stranded nucleic acid molecule, which is isolated from a natural gene or that is modified to contain nucleic acid segments in such a manner that they would otherwise not exist in nature. The expression "nucleic acid construct" is synonymous to the expression "expression cassette", when the construct of nucleic acid contains the control sequences required for the expression of the encoding sequence. Preferably, the gene construct of the invention further comprises a promoter operably linked to the nucleic acid sequence of the invention. The promoter may be, but without limitations, a constitutive or inducible promoter. As a promoter for expressing the nucleic acid sequence of the invention, a promoter which has usually been used in production of heterologous proteins in *Escherichia coli* may be used, for example, powerful promoters such as T7 promoter, trp promoter, lac promoter, tac promoter, PL promoter, can be included in the construct.

Preferably, the gene construct of the invention further comprises a terminator. Examples of such a terminator include, but without limitations, T7 terminator, fd phage terminator, T4 terminator, tetracyclin resistant gene terminator, *Escherichia coli* trpA gene terminator, and the like.

The term "expression vector", also known as "expression construct" or "plasmid", relates to a DNA molecule, linear or circular, that comprises the nucleic acid sequence of the invention operably linked to additional segments that provide the transcription of the encoded peptide. Generally, a plasmid is used to introduce a specific gene in a target cell. Once the expression vector is in the interior of the cell, the protein encoded by the gene is preferably produced by means of the ribosome complexes of the cellular transcription and translation machinery. The plasmid is often subject to engineering to contain regulating sequences that act as enhancing and promoter regions that lead to an efficient transcription of the gene carried on the expression vector. The objective of a well-designed expression vector is the production of large amounts of stable messenger RNA and, therefore, of proteins. Expression vectors are basic tools for biotechnology and for the production of proteins, such as enzymes. The expression vector of the invention is introduced in a host cell such that the vector remains as a chromosome constituent or as an extra-chromosome self-replicating vector.

The term "expression" relates to the process whereby a polypeptide is synthesised from a polynucleotide. The term includes the transcription of the polynucleotide in a messenger RNA (mRNA) and the translation of said mRNA into a protein or polypeptide.

Examples of expression vectors are, but without limitations, phages, cosmids, phagemids, yeast artificial chromosomes (YAC), bacterial artificial chromosomes (BAC), human artificial chromosomes (HAC) or viral vectors, such as adenovirus, retrovirus or lentivirus.

The appropriate expression vectors for the insertion of the polynucleotide of the invention are, preferably, plasmids used for the protein expression in prokaryotes such as, by way of example: pUC18, pUC19, Bluescript and its derivatives, mp18, mp19, pBR322, pMB9, Co1E1, pCR1, RP4, pET plasmids, phages and "launcher" vectors, such as pSA3 and pAT28; yeast expression vectors such as the 2-micron plasmid of *Saccharomyces cerevisiae,* integration plasmids, YEP vectors, centromere plasmids and similar; expression vectors in insect cells such as the vectors of the pAC and pVL series; expression vectors in plant cells such as piBi, pEarleyGate, PAVA, pCAMBIA, PGSA, PGWB, PMDC, PMY, pore series and similar, and other protein expression plasmids used in eukaryote cells, including baculovirus adequate for cell transfection.

In order to select the transformants comprising the gene construct of the invention, said gene construct preferably comprises a marker, such as an ampicillin resistant gene. Such plasmids are commercially available and have powerful promoters (pUC series (Takara S buzo), pPROK series (Clontech), pKK233-2 (Clontech), etc.). Alternatively, the marker may be other drug resistant gene such as kanamycin, neomycin or chloramphenicol resistant gene, or a fluorescence gene, such as luciferase, GFP, mCherry, and the like. Or the marker may be an auxotrophic genetic marker.

The variant of the invention can be prepared using any known means in the state of the art, such as the modification of a DNA sequence that encodes the FSA enzyme of SEQ ID NO: 1, transformation of the modified DNA sequence in an adequate host cell and the expression of the modified DNA sequence to form the mutant or variant enzyme.

Therefore, another aspect of the invention relates to a host cell comprising the nucleic acid sequence of the invention or the gene construct of the invention, hereinafter "host cell of the invention".

The term "host cell", as used in this description, relates to any prokaryote or eukaryote cell that is the recipient of an expression vector, cloning vector or any other exogenous nucleic acid molecule. Therefore, the term includes any cultivable cell that may be modified through the introduction of a nucleic acid not contained naturally therein. Preferably, a host cell is that in which the polynucleotide of the invention may be expressed, giving rise to a stable polypeptide, post-translationally modified and located in the appropriate subcellular compartment. The election of an appropriate host cell may also be influenced by the election of the detection signal. For example, the use of constructs with reporter genes (for example, *lacZ*, luciferase, thymidine kinase or green fluorescent protein "GFP") can provide a signal selectable through the activation or inhibition of the transcription of the gene of interest in response to a transcription-regulating protein. In order to achieve optimum selection or screening, the phenotype of the host cell must be considered.

The host cell of the invention may be, but without limitations, a bacterial cell, an actinomycetes cell, a yeast cell, a fungal cell, a plant cell, or an animal cell. Bacterial cells suitable for the transformation with the nucleic acid sequence of the invention or the gene construct of the invention are, for instance, bacteria of *Escherichia, Pseudomonas, Corynebacterium, Bacillus,* and the like, and *Escherichia coli* is preferably used because there are a large number of findings on the techniques for mass production of proteins using it.

The host cell of the invention may be cultivated for the production of the FSA variant of the invention. A host cell culture relates to the process of *in vitro* maintaining and growing the host cells. Cell cultures need controlled conditions of temperature, pH, proportion of gases (oxygen and carbon dioxide), etc., in addition to the presence of the adequate nutrients to permit cellular viability and division. Cell cultures can be carried out in solid substrates, such as agar, or in a liquid medium which enables the cultivation of large amounts of cells in suspension.

The culture of the host cell of the invention may be conducted in a culture medium usually employed in the art, for instance, a medium containing a carbon source, nitrogen source, inorganic salts, trace metal salts, vitamins, etc.

Thus, another aspect of the present invention refers to the use of the host cell of the invention for producing the variant of the invention.

The host cell of the invention is therefore cultured in an appropriate culture medium, and the FSA variant of the invention is accumulated in the any one of medium and cells or both.

It is possible to increase the expression of the recombinant gene in the host cell by adding to the culture medium a proper amount of an inducer responding to a promoter integrated in the gene construct of the invention. For example, when the gene construct is constructed with a *lac* promoter to which an objective gene is linked at the downstream, it is possible to add properly IPTG or alternatively it is also possible to add properly galactose.

Following cultivation, the host cells may be recovered by, for example, centrifugation. Since the majority of the FSA enzyme is present in the cells, the variant may be solubilized by disrupting or lysing the host cells. Ultrasonic disrupting, French press disrupting or glass bead disrupting may be used to disrupt the host cells. In the case of lysing the cells, a method that uses lysozyme, peptidase, or a suitable combination thereof may be adopted.

Afterwards, the variant of the invention can be purified. The term "purify", as used in the description, relates to the isolation of the variant of the invention and to its concentration, disregarding the other polypeptides present in the culture medium. The isolation or purification of the FSA variant of the invention can be carried out, for instance, using differential solubility techniques, chromatography, electrophoresis or isoelectric focusing. Chromatography techniques can be based on molecular weight, ion charge (based on the ionisation state of the amino acids under working conditions), the affinity of the protein for certain matrixes or chromatographic columns, or by means of purification tags, and can be carried out for example on a column, on paper or on a plate. The isolation or purification of the enzyme variant can be carried out, for example, by means of precipitation with ammonium sulphate, gel filtration chromatography, ion exchange chromatography, hydrophobic chromatography, hydroxyapatite chromatography, fast protein liquid chromatography (FPLC) or high performance liquid chromatography (HPLC), using automated systems that significantly reduce purification time and increase purification efficiency.

When the FSA variant of the invention derived from the host cell of the invention is purified, although the variant is purified by using an enzyme solubilizing solution for the starting material, if undisrupted or unlysed residue remains, re-centrifuging the solubilization solution and removing any residue that precipitates is advantageous to purification.

Another aspect of the invention refers to the use of the variant of the invention or the host cell of the invention as a biocatalyst for aldol carboligation reactions (*i. e.* for stereoselective C-C bond formation).

The variant of the invention may be added to the reaction medium directly or as a microorganism containing (expressing) it, as far as it catalyzes the aldol carboligation reaction. Thus, the variant of the invention may be added to the reaction, for example, as a free-enzyme, as an immobilized enzyme, as a whole cell expressing it or as an immobilized whole cell expressing it.

The term "immobilized" means that the variant of the invention or the host cell of the invention is coupled to surface, *i.e.* it is on a surface (directly linked or linked by means of any linker molecule) or the surface is coated with it. Said surface may be, for instance, a solid or semi-solid support, a matrix, a plate, a particle, preferably a nanoparticle, a gel, a biopolymer, beans or the like.

The additive comprising the variant of the invention to be added to the reaction medium may include the culture medium (the medium from which the cells have been removed), cells (including both of cultured cells and washed cells), cells processed by crushing or lysis, a composition comprising an active FSA variant of the invention derived by purification from the above-mentioned culture medium and/or cells (crude enzyme solution, purified enzyme), and the like.

For example, when the aldol carboligation reaction is produced with a whole host cell producing the variant of the invention, the substrates of the aldol reaction may be added directly to the culture broth during the incubation of the host cell. Host cells separated from the culture broth, washed cells, or cells processed by crushing or lysis may be added to the reaction mixture, or alternatively the FSA variant of the invention is recovered from the processed cells and may be added as a crude enzyme solution or purified enzyme to the reaction mixture. That is, any form of fraction comprising the variant of the invention may be used in an aldol carboligation reaction according to the invention.

In a preferred embodiment, the aldol carboligation reaction takes place between two aldehydes or between an aldehyde and a ketone.

More preferably, the aldehydes are selected from the list consisting of: 2-chloroethanal (or chloroacetaldehyde), formaldehyde, benzyloxyethanal, propanal, pentanal, isopentanal, ethanal (or acetaldehyde), butanal, an hydroxyaldehyde or a N-Cbz-aminoaldehyde. Examples of hydroxyaldehyde are 3-hydroxypropanal, R-3-hydroxybutanal or S-3-hydroxybutanal. Examples of N-Cbz-aminoaldehyde are N-Cbz-3-aminopropanal or N-Cbz-glycinal.

In another preferred embodiment, the ketone is selected from the list consisting of: propanone, butanone, cyclobutanone, cyclopentanone or 3-pentanone.

Another aspect of the invention refers to a method for aldol carboligation, hereinafter "the method of the invention", comprising the following steps:

a. reacting two aldehydes or an aldehyde and a ketone in the presence of the variant of the invention or the host cell of the invention, and

b. recovering the product of the reaction performed in step (a).

In a preferred embodiment of the method of the invention, the reaction in step (a) takes place in an aqueous buffered solution at 25°C or less.

In another preferred embodiment of the method of the invention, the product of the reaction performed in step (a) is recovered in step (b) by means of one of the following methods: column chromatography on silica or on hydrophobic polymeric support, or on activated charcoal or on a mixture of diatomaceous earth and activated charcoal or using preparative normal or reverse phase HPLC or liquid-liquid extraction, or any combination thereof.

More preferably, the aldehydes in the method of the invention are selected from the list consisting of: 2-chloroethanal (or chloroacetaldehyde), formaldehyde, benzyloxyethanal, propanal, pentanal, isopentanal, ethanal (or acetaldehyde), butanal, an hydroxyaldehyde or a N-Cbz-aminoaldehyde. Examples of hydroxyaldehyde are 3-hydroxypropanal, R-3-hydroxybutanal or S-3-hydroxybutanal. Examples of N-Cbz-aminoaldehyde are N-Cbz-3-aminopropanal or N-Cbz-glycinal.

In another preferred embodiment, the ketone in the method of the invention is selected from the list consisting of: propanone, butanone, cyclobutanone, cyclopentanone or 3-pentanone.

In a particular embodiment of the present invention, the aldehyde is 3-hydroxypropanal, the ketone is propanone and the variant of the invention comprises the amino acid substitution D6H.

In another particular embodiment, the aldehyde is S-3-hydroxybutanal, the ketone is propanone and the variant of the invention comprises the amino acid substitution D6H.

In another particular embodiment, the aldehyde is R-3-hydroxybutanal, the ketone is propanone and the variant of the invention comprises the amino acid substitution D6H.

In another particular embodiment, the aldehyde is benzyloxyethanal, the ketone is propanone and the variant of the invention comprises the amino acid substitution D6Q.

In another particular embodiment, the aldehyde is S-3-hydroxybutanal, the ketone is cyclobutanone and the variant of the invention comprises the amino acid substitution D6H.

In another particular embodiment, the aldehyde is S-3-hydroxybutanal, the ketone is cyclopentanone and the variant of the invention comprises the amino acid substitution D6H.

In another particular embodiment, the aldehyde is benzyloxyethanal, the ketone is cyclobutanone and the variant of the invention comprises the amino acid substitution D6Q.

In another particular embodiment, the aldehyde is N-Cbz-3-aminopropanal, the ketone is propanone and the variant of the invention comprises the amino acid substitution D6N.

In another particular embodiment, the aldehyde is N-Cbz-glycinal, the ketone is propanone and the variant of the invention comprises the amino acid substitution D6T.

In another particular embodiment, the aldehyde is N-Cbz-3-aminopropanal, the ketone is butanone and the variant of the invention comprises the amino acid substitution D6E.

In another particular embodiment, the aldehyde is N-Cbz-glycinal, the ketone is butanone and the variant of the invention comprises the amino acid substitution D6T.

In another particular embodiment, the aldehyde is N-Cbz-3-aminopropanal, the ketone is 3-pentanone and the variant of the invention comprises the amino acid substitution D6E.

In another particular embodiment, the aldehyde is N-Cbz-glycinal, the ketone is 3-pentanone and the variant of the invention comprises the amino acid substitution D6T.

In another particular embodiment, the aldehyde is N-Cbz-3-aminopropanal, the ketone is cyclobutanone and the variant of the invention comprises the amino acid substitution D6L.

In another particular embodiment, the aldehyde is N-Cbz-3-aminopropanal, the ketone is cyclopentanone and the variant of the invention comprises the amino acid substitution D6L.

In another particular embodiment, the aldehyde is N-Cbz-glycinal, the ketone is cyclopentanone and the variant of the invention comprises the amino acid substitution D6Q.

Variants of the invention are fully selective for the single addition (selective mono-addition) of two aldehydes (more than 99.99% selectivity in the single addition).

Thus, in another particular embodiment, the aldol carboligation reaction takes place between two aldehydes, wherein one of the aldehydes is ethanal, the other aldehyde is 3-hydroxypropanal, and the variant of the invention comprises the amino acid substitutions D6L and A165G.

In another particular embodiment, the aldol carboligation reaction takes place between two aldehydes, wherein one of the aldehydes is ethanal, the other aldehyde is S-3-hydroxybutanal, and the variant of the invention comprises the amino acid substitution D6H.

In another particular embodiment, the aldol carboligation reaction takes place between two aldehydes, wherein one of the aldehydes is ethanal, the other aldehyde is R-3-hydroxybutanal, and the variant of the invention comprises the amino acid substitution D6H.

In another particular embodiment, the aldol carboligation reaction takes place between two aldehydes, wherein one of the aldehydes is ethanal, the other aldehyde is N-Cbz-3-aminopropanal, and the variant of the invention comprises the amino acid substitutions D6E and A165G.

In another particular embodiment, the aldol carboligation reaction takes place between two aldehydes, wherein one of the aldehydes is formaldehyde, the other aldehyde is propanal, and the variant of the invention comprises the amino acid substitution D6Q.

In another particular embodiment, the aldol carboligation reaction takes place between two aldehydes, wherein one of the aldehydes is ethanal, the other aldehyde is 2-chloroethanal, and the variant of the invention comprises the amino acid substitution D6N or D6H.

In another particular embodiment, the aldehyde is propanal, the ketone is propanone and the variant of the invention comprises the amino acid substitution D6E.

In another particular embodiment, the aldehyde is butanal, the ketone is propanone and the variant of the invention comprises the amino acid substitution D6E.

In another particular embodiment, the aldehyde is pentanal, the ketone is propanone and the variant of the invention comprises the amino acid substitution D6E.

In another particular embodiment, the aldehyde is isopentanal, the ketone is propanone and the variant of the invention comprises the amino acid substitution D6E.

In another particular embodiment, the aldol carboligation reaction takes place between two aldehydes, wherein both aldehydes are propanal and the variant of the invention comprises the amino acid substitutions D6A and T26I.

In another particular embodiment, the aldol carboligation reaction takes place between two aldehydes, wherein both aldehydes are butanal and the variant of the invention comprises the amino acid substitutions D6A and T26L.

In one of the most preferred embodiments of the present invention, the aldehyde is N-Cbz-3-aminopropanal, the ketone is propanone and the variant of the invention comprises the amino acid substitution D6N.

In other of the most preferred embodiments of the present invention, the aldehyde is 3-hydroxypropanal, the ketone is propanone and the variant of the invention comprises the amino acid substitution D6H.

In another preferred embodiment of the method of the invention, this method further comprises an additional step c), at the end of the method, comprising a cyclation reaction of the product obtained in step (b). More preferably, this cyclation reaction comprises a reductive amination when the product obtained in step (b) comprises an amine group. Alternatively, this additional step of the method of the invention may be a step a'), taking place between steps (a) and (b).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and sequence listing are provided by way of illustration and are not intended to be limiting of the present invention.

## EXAMPLES

The following examples show illustrative reactions catalyzed by the FSA variants of the invention.

### Example 1. General procedure for additions of propanone (1a) to hydroxyaldehydes.

Reactions were conducted in a 50 mL screw capped conical-bottom polypropylene tubes. *Method 1*. To an enzyme (3 mg $mL^{-1}$) solution in plain water (12.8 mL), 1 M triethanolamine buffer, pH 8 (1 mL) was added. To this

solution, aldehyde (80 mM, 3.2 mL of 500 mM aqueous stock solution) and propanone (3 mL) were added. *Method 2.* To an enzyme solution (3 mg $mL^{-1}$) in plain water (16 mL), 1 M triethanolamine buffer, pH 8 (1 mL) was added. To this solution, benzyloxyethanal (80mM) dissolved in propanone (3 mL) was added. For both Methods: Total reaction volume was 20 mL. Reaction mixtures were shaken (1000 rpm) at 25 °C for 24 h. After that, the reactions were stopped with MeOH (30 mL) to precipitate the enzyme. *Method 1*. The mixture was filtered through Celite, the MeOH was evaporated and the aqueous residue was lyophilized. *Method 2.* The mixture was filtered through activated charcoal. MeOH was evaporated and the reaction was lyophilized.

**Example 1.1.**

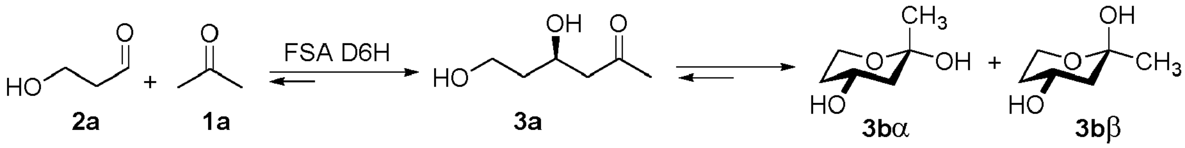


2a 1a 3a 3bα 3bβ

**2-Methyltetrahydro-2*H*-pyran-2,4-diol (3bα=(2*S*,4*R*), 3bβ=(2*R*,4*R*) and (*R*)-4,6-dihydroxyhexan-2-one (3a):** This compounds were obtained following the procedure described above (Method 1) as a mixture of anomers (**3b**α and **3b**β) and in equilibrium (slow) with the acyclic precursor (**3**). After enough time it can be found **3b**β as major product. 3-Hydroxypropanal (**2a**) (1.6 mmol) and FSA D6H were used. The product was purified by silica gel column chromatography and eluted with a step gradient of $CHCl_3$:MeOH from 1:0 to 93:7, yielding 50.0 mg (32%) of a mixture (**3b**α:**3b**β**:3**. $[\alpha]_D^{20}$= - 24.2 (c = 1.07 in MeOH); (**3b**α): $^1H$ NMR (500 MHz, $CD_3OD$) δ 3.94 (m, 2H), 3.66 (ddd, *J* = 11.6, 5.3, 1.8 Hz, 1 H), 3.58 (ddd, *J* = 12.8, 11.4, 2.3 Hz, 1 H), 2.05 (ddd, *J* = 12.6, 4.8, 2.1 Hz, 1 H), 1.82 (ddt, *J* = 10.4, 4.6, 2.2 Hz, 1 H), 1.41 (m, 1 H), 1.37 (s, 3H), 1.28 (m, 1 H). $^{13}C$ NMR (126 MHz, $CD_3OD$) δ 100.8, 65.0, 60.6, 45.9, 35.6, 23.9. (**3b**β): $^1H$ NMR (400 MHz, $CD_3OD$) δ 3.99 (m, 1 H), 3.56 (m, 1 H), 3.49 (m, 1 H), 2.09 (m, 1 H), 2.06 (m, 1 H), 1.71 (m, 1 H), 1.65 (m, 1 H), 1.33 (s, 3H). $^{13}C$ NMR (126 MHz, $CD_3OD$) δ 96.3, 65.1, 57.8, 46.0, 38.3, 24.3. Open form **3**: $^1H$ NMR (500 MHz, $CD_3OD$) δ 4.25-4.12 (m, 1 H), 4.00 - 3.85 (m, 2H), 2.61 (dd, *J* = 6.4, 2.8 Hz, 2H), 2.18 (s, 3H), 1.74-1.62 (m, 2H).$^{13}C$ NMR (101 MHz, $CD_3OD$) δ 210.5, 66.4, 59.8, 51.9, 40.6, 30.6. ESI-TOF: calculated for $[M+Na]^+$ $C_6H_{12}O_3Na$: 155.0684, found 155.0689.

**Example 1.2.**

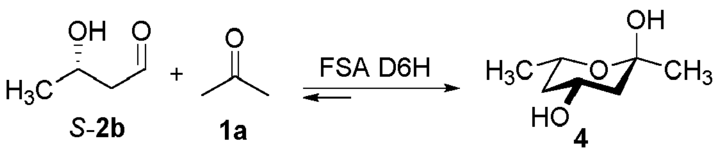


*S*-2b 1a 4

**(2*R*,4*R*,6*S*)-2,6-dimethyltetrahydro-2*H*-pyran-2,4-diol** (**4**): This compound was obtained following the procedure described above (Method 1). S-3-Hydroxybutanal (***S*-2b**) (1.6 mmol) and FSA D6H were used. The product was purified by silica gel column chromatography and eluted with a step gradient of $CHCl_3$:MeOH from 1:0 to 95:5, yielding 60.7 mg (25%) of **4.** $[\alpha]_D^{20}$= - 58.8 (*c* = 1.12 in MeOH); $^1H$ NMR (400 MHz, $CD_3OD$) δ 3.96 (tt, *J* = 11.2, 4.7 Hz, 1 H), 3.67 (ddd, *J* = 11.5, 6.4, 2.1 Hz, 1 H), 2.03 (ddd, *J* = 12.5, 4.8, 1.9 Hz, 1 H), 1.89 (ddt, *J* = 12.3, 4.4, 2.1 Hz, 1 H), 1.29 (s, 3H), 1.25 (dd, *J* = 12.5, 11.2 Hz, 1 H), 1.17 (d, *J* = 6.3 Hz, 3H), 1.05 (dd, *J* = 12.3, 11.4 Hz, 1H). $^{13}C$ NMR (101 MHz, $CD_3OD$) δ 66.5, 65.2, 45.3, 43.0, 24.1, 21.7. ESI-TOF: calculated for $[M+Na]^+$ $C_7H_{14}O_3Na$: 169.0840, found 169.0837.

**Example 1.3.**

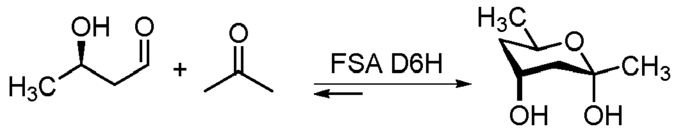


*R*-**2b** **1a** **5**

**(*2S,4R,6R*)-2,6-dimethyltetrahydro-2*H*-pyran-2,4-diol** (**5**): This compound was obtained following the procedure described above (Method 1). (*R*)-3-Hydroxybutanal (***R*-2b**) (1.6 mmol) and FSA D6H were used. The product was purified by silica gel column chromatography and eluted with a step gradient of $CHCl_3$:MeOH from 1:0 to 95:5, yielding 21.2 mg (10%) of **5.** $[\alpha]_D^{20}$= + 11.1 (*c* = 0.36 in MeOH); $^1H$ NMR (500 MHz, $CD_3OD$) δ 4.03 (m, 1H), 4.04 (m, 1 H), 1.93 (ddd, *J* = 14.4, 2.9, 2.1 Hz, 1 H), 1.73 (ddt, *J* = 13.7, 3.2, 2.2 Hz, 1 H), 1.70 - 1.65 (m, 1 H), 1.48 - 1.39 (m, 1 H), 1.30 (s, 3H), 1.20 (d, *J* = 6.3 Hz, 3H). $^{13}C$ NMR (101 MHz, $CD_3OD$) δ 100.0, 64.7, 60.8, 39.8, 38.8, 22.9, 20.1. ESI-TOF: calculated for $[M+Na]^+$ $C_7H_{14}O_3Na$: 169.0840, found 169.0842.

**Example 1.4.**

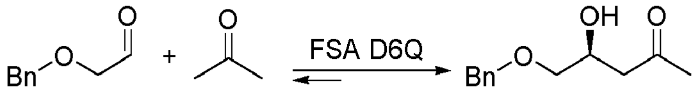


**2c** **1a** **6**

**5-(benzyloxy)-4-hydroxypentan-2-one (6):** This compound was obtained following the procedure described above (Method 2). Benzyloxyethanal **(2c)** (240 μL, 1.6 mmol) and FSA D6Q were used. After 24 h an additional amount of FSA D6Q (3 mg $ml^{-1}$) was added and the reaction was shaken (1000 rpm) at 25 °C for 24 h more. The product was purified by silica gel column chromatography and eluted with a step gradient of hexane:AcOEt from 1:0 to 1:1, yielding 256 mg (77%) of **6.** $[\alpha]_D^{20}$= - 5.29 (c = 1.26 in MeOH); HPLC: 10 to 100% of B in 30 min, $t_R$ = 16 min; $^1H$ NMR (400 MHz, $CD_3OD$) δ 7.45 - 7.10 (m, 5H), 4.53 (d, *J* = 1.9 Hz, 2H), 4.27 - 4.17 (m, 1 H), 3.50 - 3.37 (m, 2H), 2.72 - 2.54 (m, 2H), 2.16 (s, 3H). $^{13}C$ NMR (101 MHz, $CD_3OD$) δ 210.1, 138.4, 127.9, 127.4, 127.2, 75.0, 74.3, 67.8, 49.6, 30.7.

**Example 2. Scale up of aldol addition of cyclobutanone and cyclopentanone to hydroxyaldehydes.**

General procedure:

The reaction was conducted in a 50 mL screw capped conical-bottom polypropylene tubes. *Method 1.* To an enzyme (3 mg $mL^{-1}$) solution in plain water (14.8 mL), 1 M triethanolamine buffer, pH 8 (1 mL) was added. To this solution, the aldehyde (80 mM, 3.2 mL of 500 mM aqueous stock solution) and ketone (1 mL) were added. *Method 2.* To an enzyme (3 mg $mL^{-1}$) solution in plain water (18 mL), 1 M triethanolamine buffer, pH 8 (1 mL) was added. To this solution, aldehyde (80 mM) and ketone (1 mL) were added. *Methods 1* and 2: Total reaction volume was 20 mL. Reaction mixtures were shaken (1000 rpm) at 25 °C for 24 h. After that, the reaction was stopped with MeOH (30 mL) to precipitate the enzyme. *Method 1*: the mixture was filtered through activated charcoal. *Method 2* the mixture was filtered through Celite. *Methods 1* and 2: MeOH was evaporated and the reaction was lyophilized.

**Example 2.1.**

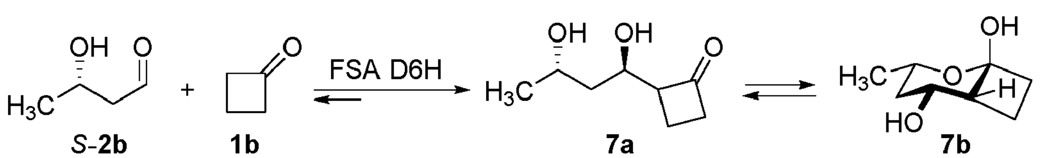


*S*-**2b** **1b** **7a** **7b**

**(1*R*,3*S*,5*R*,6*R*)-3-methyl-2-oxabicyclo[4.2.0]octane-1,5-diol (7b) in equilibrium with its acyclic form 7a:** This compound was obtained following the procedure described above (Method 1). (*S*)-3-Hydroxybutanal (*S*-**2b**) (1.6 mmol), cyclobutanone (**1b**) and FSA D6H were used. The product was purified by silica gel column chromatography

and eluted with a step gradient of $CHCl_3$:MeOH from 1:0 to 95:5, yielding 55.3 mg (22%) of **7b.** $[\alpha]_D^{20}$= + 13.69 (c = 2.0 in MeOH); $^1$H NMR (400 MHz, $CD_3OD$) δ 3.93 (dt, *J* = 12.0, 6.3 Hz, 1 H), 3.72 (dqd, *J* = 11.1, 6.3, 1.4 Hz, 1 H), 2.65 (ddd, *J* = 9.9, 8.9, 6.5 Hz, 1 H), 1.96 - 1.89 (m, 2H), 1.73 (dddd, *J* = 12.7, 5.9, 1.5, 0.7 Hz, 1 H), 1.64 - 1.53 (m, 2H), 1.36 (dd, *J* = 12.6, 11.4 Hz, 1 H), 1.20 (d, *J* = 6.3 Hz, 3H). $^{13}$C NMR (101 MHz, $CD_3OD$) δ 67.6, 65.7, 46.8, 38.3, 34.8, 21.8, 13.3. **(7a) (*R*)-2-((1*R*,3*S*)-1,3-dihydroxybutyl)cyclobutan-1-one:** $^1$H NMR (400 MHz, $CD_3OD$) δ 3.94 (m, 2H), 3.39 (dddd, *J* = 9.7, 7.1, 5.3, 2.7 Hz, 1 H), 3.02 - 2.80 (m, 2H), 2.18 - 2.04 (m, 1 H), 2.02 - 1.95 (m, 1 H), 1.71 (m, 1 H), 1.55 - 1.48 (m, 1H) 1.18(d, *J* = 1.7 Hz, 3H). $^{13}$C NMR (101 MHz, $CD_3OD$) δ 68.4, 67.5, 65.1, 45.6, 45.1, 24.3, 14.3. ESI-TOF: calculated for $[M+Na]^+$ $C_8H_{14}O_3Na$: 181.0840, found 181.0843.

**Example 2.2.**

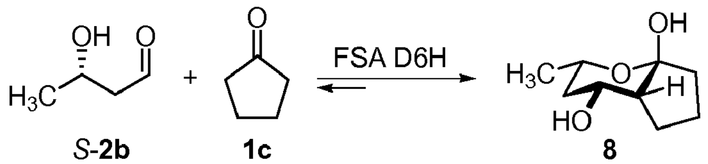


*S*-**2b** **1c** **8**

**(2*S*,4*R*,4a*R*,7a*R*)-2-methylhexahydrocyclopenta[b]pyran-4,7a(2H)-diol (8):** This compound was obtained following the procedure described above (Method 1). (*S*)-3-Hydroxybutanal **(*S*-2b)** (1.6 mmol), cyclopentanone (**1c**) and FSA D6H were used. The product was purified by silica gel column chromatography and eluted with a step gradient of $CHCl_3$:MeOH from 1:0 to 95:5, yielding 22.6 mg (8%) of **8**. $[\alpha]_D^{20}$= -2.03 (*c* = 1.49 in MeOH); $^1$H NMR (400 MHz, $CD_3OD$) δ 4.05 (dt, *J* = 11.8, 5.4 Hz, 1 H), 3.71 (dqd, *J* = 12.7, 6.3, 2.1 Hz, 1 H), 2.20 (h, *J* = 5.6 Hz, 1 H), 1.94 - 1.86 (m, 1 H), 1.78 - 1.57 (m, 6H), 1.43 - 1.30 (m, 1H), 1.18 (d, *J* = 6.3 Hz, 3H). $^{13}$C NMR (126 MHz, $CD_3OD$) δ 109.5 (d, *J* = 9.0 Hz), 65.9, 65.6, 47.1, 35.6, 34.6, 21.6, 20.3, 19.4. ESI-TOF: calculated for $[M+Na]^+$ $C_9H_{16}O_3Na$: 195.0997, found 195.0991.

**Example 2.3.**

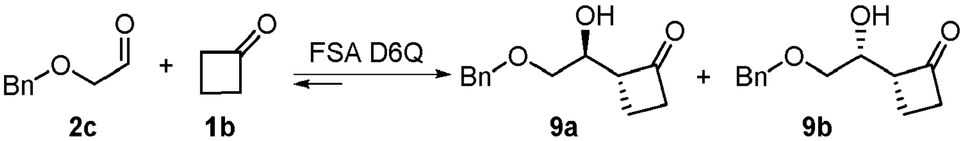


**2c** **1b** **9a** **9b**

**(*R*)-2-((*S*)-2-(benzyloxy)-1-hydroxyethyl)cyclobutan-1-one (9a) (75%).** This compound was obtained following the procedure described above (Method 2). Benzyloxyethanal (**2c**) (1.6 mmol), cyclobutanone (**1b**) and FSA D6Q were used. The product was purified by silica gel column chromatography and eluted with a step gradient of hexane:AcOEt from 1:0 to 3:7, yielding 46.0 mg (13 %) of product. HPLC: 10 to 100 % of B in 30 min, $t_R$ = 18 min; $^1$H NMR (400 MHz, $CD_3OD$) δ 7.38 - 7.26 (m, 5H), 4.53 (s, 2H), 3.92 (dt, *J* = 6.5, 5.0 Hz, 1 H), 3.59 (dd, *J* = 9.8, 6.6 Hz, 1 H), 3.55 (m, 1 H), 3.53 (m, 1 H), 2.93 (ddd, *J* = 10.1, 7.7, 2.7 Hz, 1 H), 2.88 (ddd, *J* = 9.8, 6.2, 2.5 Hz, 1 H), 2.15 - 1.96 (m, 2H). $^{13}$C NMR (101 MHz, $CD_3OD$) δ 212.0, 139.6, 129.3, 128.9, 128.7, 74.3, 73.5, 70.3, 64.1, 45.8, 13.9. Minor **(*R*)-2-((*R*)-2-(benzyloxy)-1-hydroxyethyl)cyclobutan-1-one (9b) (25%):** $^1$H NMR (400 MHz, $CD_3OD$) δ 7.38-7.26 (m, 5H), 4.53 (s, 2H), 4.05 (dt, *J* = 6.3, 5.0 Hz, 1 H), 3.47 (dd, *J* = 9.0, 5.2, 1 H), 3.40 (dd, *J* = 9.8, 6.3, 1 H), 3.05 (m, 1 H), 2.81 (m, 1 H), 2.15-1.96 (m, 2H). $^{13}$C NMR (101 MHz, $CD_3OD$) δ 212.3, 139.6, 129.3, 128.9, 128.7, 74.3, 73.6, 69.1, 64.3, 46.0, 13.1. ESI-TOF: calculated for $[M+Na]^+$ $d_{13}H_{16}O_3Na$: 243.0997, found 243.0990.

**Example 3. Scale up of aldol addition of ethanal (1d) to hydroxyaldehydes.**

General procedure:

The reaction was conducted in a 50 mL screw capped conical-bottom polypropylene tubes. To an enzyme (3 mg $mL^{-1}$) solution in plain water (15.7 mL), 1 M triethanolamine buffer, pH 8 (1 mL) was added. To this solution, aldehyde (80 mM, 3.2 mL of 500 mM aqueous stock solution) and ethanal (5%, 1 mL, using electrophile **2a** or 100 mM, 112 μL using electrophiles **2b**) were added. Total reaction volume was 20 mL. Reaction mixtures were shaken (1000 rpm) at

25 °C for 24 h. After that, the reaction was stopped with MeOH (30 mL) to precipitate the enzyme, and the mixture was filtered through activated charcoal. MeOH was evaporated and the reaction was lyophilized.

**Example 3.1.**

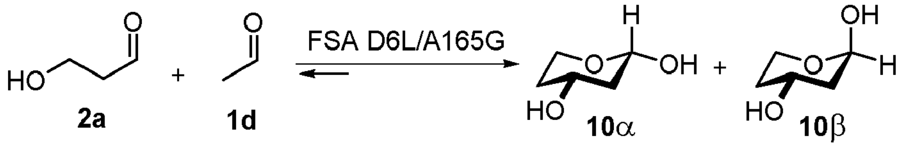


2a 1d 10α 10β

**(2*S*,4*R*)-tetrahydro-2*H*-pyran-2,4-diol (10α-anomer) and (2*R*,4*R*)-tetrahydro-2*H*-pyran-2,4-diol (7a)** (10β-anomer): These compounds were obtained as a mixture (≈ 1:1) following the procedure described above. 3-Hydroxypropanal (**2a**) (1.6 mmol) and FSA D6L/A165G were used. The product was purified by silica gel column chromatography and eluted with a step gradient of $CHCl_3$:MeOH from 1:0 to 95:5, yielding 41.5 mg (22 %) of **10**α:**10**β. $[\alpha]_D^{20}$= - 2.93 (*c* = 0.62 in MeOH). (**10**α-Anomer): $^1$H NMR (400 MHz, $CD_3OD$) δ 5.15 (dd, *J* = 4.4, 3.1 Hz, 1 H), 4.06 (dd, *J* = 8.4, 4.4 Hz, 1 H), 3.87 (dd, *J* = 8.8, 3.2 Hz, 1 H), 3.72 (dd, *J* = 5.6, 4.3 Hz, 1 H), 1.85 - 1.76 (m, 2H), 1.58 (ddd, *J* = 12.9, 8.5, 3.1 Hz, 1 H), 1.49 (ddd, *J* = 12.9, 8.6, 4.1 Hz, 1H). $^{13}$C NMR (101 MHz, $CD_3OD$) δ 91.9, 63.1, 58.6, 39.6, 33.9. (**10**β-Anomer): $^1$H NMR (400 MHz, $CD_3OD$) δ 4.61 (dd, *J* = 9.0, 2.2 Hz, 1 H), 3.98 - 3.93 (m, 1 H), 3.72 (m, 1 H), 3.40 (td, *J* = 11.9, 2.5 Hz, 1 H), 2.07 (ddt, *J* = 12.1, 4.2, 2.0 Hz, 1 H), 1.77 (m, 1 H), 1.39 (m, 1 H), 1.29 (m, 1H). $^{13}$C NMR (101 MHz, $CD_3OD$) δ 94.2, 66.0, 61.1, 41.8, 34.1. ESI-TOF: calculated for $[M+2Na]^+$ $C_5H_{10}O_3Na_2$: 165.0503, found 165.0507.

**Example 3.2.**

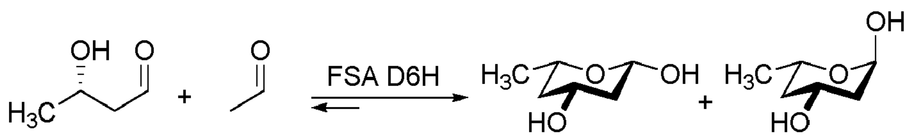


*S*-**2b** **1d** **11**α **11**β

**(2*S*,4*R*,6*S*)-6-methyltetrahydro-2*H*-pyran-2,4-diol (11α-anomer) (2*R*,4*R*,6*S*)-6-methyltetrahydro-2*H*-pyran-2,4-diol (11β-anomer).** These compounds were obtained as a mixture (≈ 1:1) following the procedure described above. *S*-3-Hydroxybutanal **(*S*-2b)** (1.6 mmol) and FSA D6H were used. The product was purified by silica gel column chromatography and eluted with a step gradient of $CHCl_3$:MeOH from 1:0 to 95:5, yielding 57.1 mg (27%) of **11**α**:11**β. $[\alpha]_D^{20}$= - 15.68 (*c* = 0.19 in MeOH). (**11**α-anomer): $^1$H NMR (400 MHz, $CD_3OD$) δ 4.65 (dd, *J* = 9.7, 2.1 Hz, 1 H), 3.80 - 3.70 (m, 1 H), 3.52 (ddd, *J* = 11.2, 6.3, 2.0 Hz, 1 H), 2.09 (ddt, *J* = 12.0, 4.3, 2.0 Hz, 1 H), 1.86 (ddt, *J* = 12.4, 4.2, 1.9 Hz, 1 H), 1.22 (d, *J* = 6.2 Hz, 3H), 1.12 - 1.00 (m, 2H). $^{13}$C NMR (101 MHz, $CD_3OD$) δ 92.0, 67.9, 66.0, 41.7, 41.6, 20.2. (**11**β-anomer): $^1$H NMR (400 MHz, $CD_3OD$) δ 5.29 (d, *J* = 3.6 Hz, 1 H), 4.17 - 3.98 (m, 2H), 2.02 - 1.90 (m, 3H), 1.40 (ddd, *J* = 12.4, 11.4, 3.6 Hz, 1 H), 1.16 (d, *J* = 6.3 Hz, 3H). $^{13}$C NMR (101 MHz, $CD_3OD$) δ 93.9, 63.5, 62.6, 42.5, 39.2, 20.4. ESI-TOF: calculated for $[M+H]^+$ $C_6H_{13}O_3$: 133.0864, found 133.0860.

**Example 3.3.**

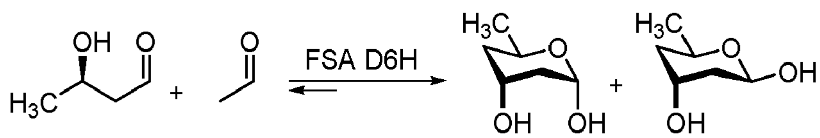


*R*-**2b** **1d** **12**α **12**β

**(2*S*,4*R*,6*R*)-6-methyltetrahydro-2*H*-pyran-2,4-diol** (12α-anomer) **(2*R*,4*R*,6*R*)-6-methyltetrahydro-**

**2*H*-pyran-2,4-diol** (**12**β-anomer). These compounds were obtained as a mixture of α:β anomers ≈ 4:1 following the procedure described above. R-3-Hydroxybutanal (*R*-**2b**) (1.6 mmol) and FSA D6H were used. The product was purified by silica gel column chromatography and eluted with a step gradient of $CHCl_3$:MeOH from 1:0 to 95:5, yielding 28.7 mg (14%) of product **12**α:**12**β. $[\alpha]_D^{20}$ = - 8.5 (*c* = 1.7 in MeOH);(**12**α-anomer) $^1H$ NMR (400 MHz, $CD_3OD$) δ 5.02 (dd, *J* = 9.9, 2.2 Hz, 1H), 4.17 (t, *J* = 3.0 Hz, 1 H), 3.99 (dqd, *J* = 12.6, 6.3, 2.2 Hz, 1 H), 1.83 (ddt, *J* = 13.5, 3.1, 2.1 Hz, 1 H), 1.63 - 1.55 (m, 1 H), 1.43 (ddd, *J* = 13.3, 9.9, 3.0 Hz, 1 H), 1.38 - 1.30 (m, 1 H), 1.15 (d, *J* = 6.3 Hz, 3H). $^{13}C$ NMR (101 MHz, $CD_3OD$) δ 91.7, 66.4, 64.8, 39.0, 39.0, 20.3. (**12**β-anomer) $^1H$ NMR (400 MHz, $CD_3OD$) δ 5.14 (t, *J* = 3.0 Hz, 1 H), 4.36 (ddd, *J* = 10.8, 6.5, 2.8 Hz, 1 H), 4.11 - 4.08 (m, 1 H), 1.77 (td, *J* = 3.4, 2.9, 1.4 Hz, 1 H), 1.76 - 1.69 (m, 1 H), 1.53 - 1.48 (m, 1 H), 1.15 (d, *J* = 6.3 Hz, 3H). $^{13}C$ NMR (101 MHz, $CD_3OD$) δ 92.1, 64.2, 59.5, 39.3, 35.5, 20.1. ESI-TOF: calculated for $[M+H]^+$ $C_6H_{13}O_3$: 133.0864, found 133.0865.

**Example 4. Scale up of aldol addition of propanone, 2-butanone, 3-pentanone, ciclobutanone and cyclopentanone to *N*-Cbz-aminoaldehydes.**

General procedure:

The reaction was conducted in a 50 mL screw capped conical-bottom polypropylene tubes (Falcon tubes). To an enzyme solution (3 mg $mL^{-1}$) in plain water (16 mL or 18 mL), 1 M triethanolamine buffer pH 8 (1 mL) was added. To this solution, the aldehyde (80 mM) dissolved in the ketones (3 mL acetone, 1 mL the rest) was added. Total reaction volume was 20 mL. Reaction mixtures were shaken (1000 rpm) at 25 °C for 24 h. After that, the reaction was stopped with MeOH (30 mL) to precipitate the enzyme. Then, the mixture was filtered through Celite, the MeOH was evaporated and the aqueous residue was lyophilized, purified characterized and submitted to reductive amination.

Intramolecular reductive amination

General procedure: The aldol adduct (3 mM final concentration) was dissolved in $H_2O$:MeOH 1:1 (70 mL), then Pd/C (60 mg) was added. The mixture was shaken under $H_2$ (2.5 atm) overnight at room temperature. After that, the reaction was filtered and the solvent was evaporated. Products were characterized without any further purification.

**Example 4.1.**

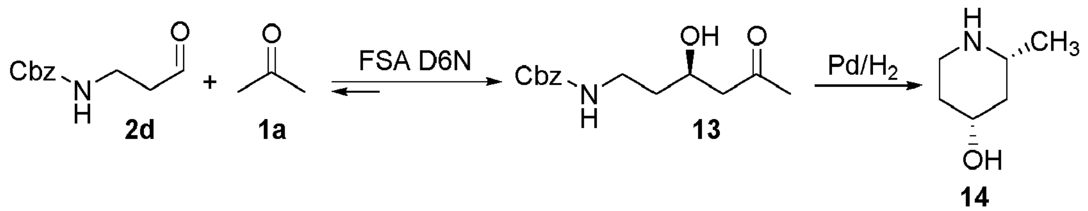


**(*R*)-*N*-Cbz-6-amino-4-hydroxyhexan-2-one (13):** This compound was obtained following the procedure described above. *N*-Cbz-3-aminopropanal (**2d**) (331.2 mg, 1.6 mmol) and FSA D6N were used. After 24 h an additional amount of FSA D6N (3 mg $ml^{-1}$) was added and the reaction was shaken (1000 rpm) at 25°C for 24 h. The product was purified by silica gel column chromatography and eluted with a step gradient of hexane:AcOEt from 1:0 to 3:7, yielding 266.2 mg (85%) of the title compound. $[\alpha]_D^{20}$= + 10.74 (c = 2.1 in MeOH); HPLC: 10 to 100% of B in 30 min, $t_R$ = 16 min; Chiral HPCL: $t_R$ = 25 min (racemic sample: $t_R(R)$ = 25 min, $t_R(S)$ = 30 min); $^1H$ NMR (400 MHz, $CD_3OD$) δ 7.45 - 7.18 (m, 5H), 5.07 (s, 2H), 4.19 - 3.99 (m, 1 H), 3.23 (t, *J* = 7.0 Hz, 2H), 2.59 (d, *J* = 6.4 Hz, 2H), 2.15 (s, 3H), 1.76 - 1.47 (m, 2H). $^{13}C$ NMR (101 MHz, $CD_3OD$) δ 210.4, 159.0, 138.4, 129.4, 128.9, 128.8, 67.4, 66.5, 51.7, 38.6, 38.2, 30.6.

The intramolecular reductive amination reaction produced: **(2*R*,4*R*)-2-methylpiperidin-4-ol (14):** $^1H$ NMR (400 MHz, $CD_3OD$) δ 3.77 (tt, *J* = 11.0, 4.5 Hz, 1H), 3.41 - 3.33 (m, 1 H), 3.25 - 3.14 (m, 1 H), 2.97 (td, *J* = 13.4, 3.0 Hz, 1 H), 2.09 (tdd, *J* = 13.3, 4.7, 2.5 Hz, 2H), 1.55 (tdd, *J* = 13.7, 11.0, 4.5 Hz, 1 H), 1.39 (dd, *J* = 13.1, 11.3 Hz, 1 H), 1.32 (d, *J* = 6.6 Hz, 3H). $^{13}C$ NMR (101 MHz, $CD_3OD$) δ 65.2, 51.4, 42.3, 39.5, 30.9, 18.0. ESI-TOF: calculated for $[M+H]^+$ $C_6H_{14}NO$: 116.1075, found 116.1077.

**Example 4.2.**

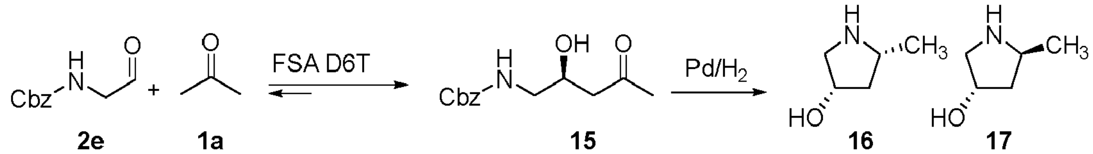


2e 1a 15 16 17

**(*S*)-*N*-Cbz-5-amino-4-hydroxypentan-2-one (15):** This compound was obtained following the procedure described above. *N*-Cbz-glycinal **(2e)** (310.4 mg, 1.6 mmol) and FSA D6T were used. The product was purified by silica gel column chromatography and eluted with a step gradient of Hexane:AcOEt from 1:0 to 3:7, yielding 114.8 mg (29%) of product. $[\alpha]_D^{20}$= + 3.4 (c = 1.9 in MeOH); HPLC:10 to 100 % of B in 30 min, $t_R$ = 15 min; Chiral HPCL: $t_R$ = 31 min (racemic sample: $t_R(R)$ = 31 min, $t_R(S)$ = 28 min); $^1$H NMR (400 MHz, $CD_3OD$) δ 7.34 (d, *J* = 6.0 Hz, 5H), 5.08 (s, 2H), 4.18 - 4.04 (m, 1 H), 3.16 (dd, *J* = 5.8, 1.4 Hz, 2H), 2.64 - 2.52 (m, 2H), 2.15 (s, 3H). $^{13}$C NMR (101 MHz, $CD_3OD$) δ 209.9, 159.1, 138.3, 129.4, 129.0, 128.8, 126.3, 68.0, 67.5, 48.4, 47.4, 30.6.

The intramolecular reductive amination reaction produced two diastereoisomers in a 70:30 ratio. Major: **(3*S*,5*R*)-5-methylpyrrolidin-3-ol (16).** $^1$H NMR (400 MHz, $CD_3OD$) δ 4.41 - 4.31 (m, 1 H), 3.22 (p, *J* = 7.0, 7.0, 6.9, 6.9 Hz, 1 H), 2.92 (dd, *J* = 12.2, 1.5 Hz, 1 H), 2.87 (dd, *J* = 12.2, 4.9 Hz, 1H), 2.27 (ddd, *J* = 13.5, 7.8, 6.5 Hz, 1 H), 1.32 (dddd, *J* = 13.6, 7.8, 3.7, 1.2 Hz, 1H), 1.27 (d, *J* = 6.5 Hz, 3H). $^{13}$C NMR (101 MHz, $CD_3OD$) δ 71.8, 54.5, 54.0, 42.1, 19.3. Minor: **(3*S*,5*S*)-5-methylpyrrolidin-3-ol (17).** $^1$H NMR (400 MHz, $CD_3OD$) δ 4.41 - 4.29 (m, 1 H), 3.45 (dp, *J* = 10.0, 6.5, 6.5, 6.4, 6.4 Hz, 1 H), 3.27 (dd, *J* = 12.1, 5.2 Hz, 1 H), 2.83 - 2.74 (m, 1 H), 1.92 (ddt, *J* = 13.4, 6.2, 1.4, 1.4 Hz, 1 H), 1.48 (ddd, *J* = 13.4, 10.0, 5.8 Hz, 1 H), 1.20 (d, *J* = 6.5 Hz, 3H). $^{13}$C NMR (101 MHz, $CD_3OD$) δ 71.5, 54.0, 52.9, 42.8, 18.3. ESI-TOF: calculated for $[M+H]^+$ $C_5H_{12}NO$: 102.0919, found 102.0210.

**Example 4.3.**

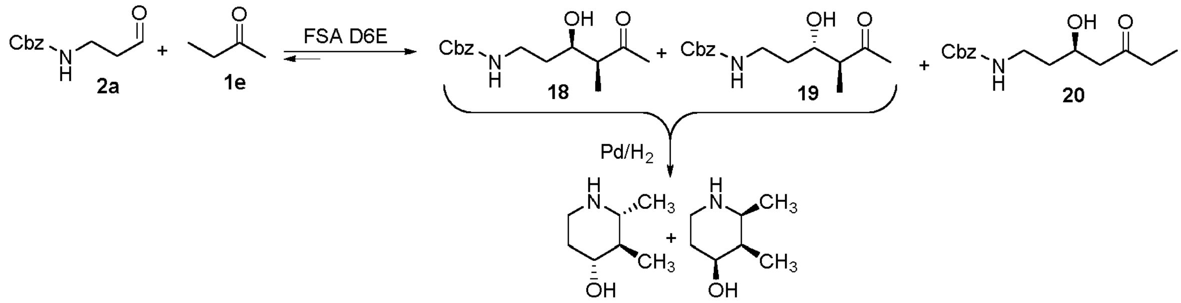


2a 1e 18 19 20 21 22

**Major regioisomers: (3*S*,4*R*)-*N*-Cbz-6-Amino-4-hydroxy-3-methylhexan-2-one (18) and (3*S*,4*S*)-*N*-Cbz-6-Amino-4-hydroxy-3-methylhexan-2-one (19).** This compound was obtained following the procedure described above. *N*-Cbz-3-aminopropanal **(2a)** (1.6 mmol), butanone **(1e)** and FSA D6E were used. The product was purified by silica gel column chromatography and eluted with a step gradient of hexane:AcOEt from 1:0 to 3:7, yielding 213 mg (48%) of **18+19,** although **19** could not be assigned in the NMR spectra and the percentage formed during the aldol addition was inferred from the products obtained in the reductive amination. $[\alpha]_D^{20}$= + 33.5 (*c* = 2.1 in MeOH); HPLC: 10 to 100 % of B in 30 min, $t_R$ = 18 min; $^1$H NMR (400 MHz, $CD_3OD$) δ 7.50 - 7.19 (m, 5H), 5.05 (s, 2H), 3.90 (dt, *J* = 9.2, 4.5 Hz, 1 H), 3.26 - 3.15 (m, 2H), 2.67 - 2.53 (m, 1 H), 2.14 (s, 3H), 1.57 (tdd, *J* = 15.2, 7.7, 5.1 Hz, 2H), 1.06 (d, *J* = 7.0 Hz, 3H). $^{13}$C NMR (101 MHz, $CD_3OD$) δ 214.0, 158.2, 137.1, 128.7, 128.3, 128.1, 69.0, 65.9, 52.3, 37.3, 34.7, 28.2, 9.8. **Minor regioisomer: (*R*)-*N*-Cbz-7-amino-5-hydroxyheptan-3-one (20).** The product was purified by preparative HPLC and eluted with a step gradient of $H_2O$:$CH_3CN$ from 1:0 to 1:1, flow rate 1 mL $min^{-1}$ and detection at 215 nm, yielding 25 mg of a mixture 44:56 of regioisomers I:II. $[\alpha]_D^{20}$= + 18.5 (c = 0.9 in MeOH); HPLC: 10 to 100% of B in 30 min, $t_R$ = 19 min; $^1$H NMR (400 MHz, $CD_3OD$) δ 7.59 - 7.17 (m, 5H), 5.07 (s, 2H), 4.09 (dt, *J* = 8.2, 4.0 Hz, 1 H), 3.24 (q, *J* = 7.0, 6.4 Hz, 2H), 2.59 - 2.54 (m, 2H), 2.49 (qd, *J* = 7.3, 1.8 Hz, 2H), 1.76 - 1.60 (m, 2H), 1.01 (t, 3H). $^{13}$C NMR (101 MHz, $CD_3OD$) δ 212.7, 159.0, 138.5, 129.2, 128.9, 128.8, 66.2, 65.3, 49.1, 36.8, 36.1, 34.6, 6.43.

The intramolecular reductive amination reaction of **regioisomers 18+19** produced two diastereoisomers, **21** and **22,** in a 87:13 ratio. Major: **(2*R*,3*R*,4*R*)-2,3-dimethylpiperidin-4-ol (21):** $^1$H NMR (400 MHz, $CD_3OD$) δ 3.36 - 3.22 (m, 2H), 2.96 (td, *J* = 13.3, 3.1 Hz, 1 H), 2.81 (dq, *J* = 10.7, 6.5 Hz, 1 H), 2.05 (ddt, *J* = 13.7, 5.2, 2.8 Hz, 1 H), 1.71 - 1.56 (m, 1 H), 1.44 - 1.32 (m, 1 H), 1.30 (d, *J* = 6.5 Hz, 3H), 1.06 (d, *J* = 6.5 Hz, 3H). $^{13}$C NMR (101 MHz, $CD_3OD$) δ 70.6, 56.3, 43.6, 42.3, 31.6, 16.3, 12.4. Minor. **(2*S*,3*R*,4*S*)-2,3-dimethylpiperidin-4-ol (22):** $^1$H NMR (500 MHz, $CD_3OD$)

δ 3.83 (dt, *J* = 10.2, 4.4 Hz, 1 H), 3.32 - 3.28 (m, 2H), 3.24 (d, *J* = 12.0 Hz, 1H), 2.91 - 2.85 (m, 1 H), 2.05 - 2.02 (m, 1 H), 1.77 - 1.66 (m, 2H), 1.25 (d, *J* = 6.8 Hz, 3H), 0.94 (d, *J* = 7.2 Hz, 3H). $^{13}C$ NMR (101 MHz, $CD_3OD$) δ 68.3, 53.8, 42.7, 37.7, 26.6, 15.1, 4.8. ESI-TOF: calculated for $[M+H]^+$ $C_7H_{16}NO$: 130.1231, found 130.1236.

**Example 4.4.**

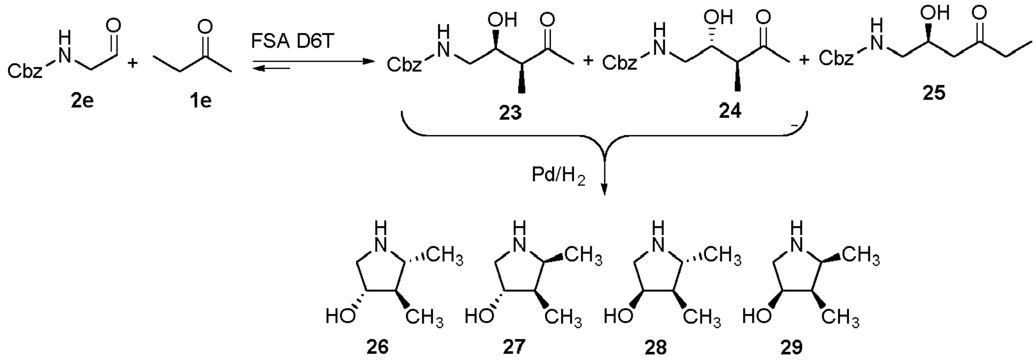


**Major regioisomers: (3*S*,4*S*)-*N*-Cbz-5-amino-4-hydroxy-3-methylpentan-2-one (23) and (3*S*,4*R*)-*N*-Cbz-5-amino-4-hydroxy-3-methylpentan-2-one (24)** (from the most substituted side of Nu). These compounds were obtained following the procedure described above. *N*-Cbz-glycinal **(1e)** (1.6 mmol), butanone and FSA D6T were used. The product was purified by silica gel column chromatography and eluted with a step gradient of hexane:AcOEt from 1:0 to 3:7, yielding 119.2 mg (28%) of **23+24.** $[\alpha]_D^{20}$= + 13.0 (*c* = 0.78 in MeOH); HPLC:10 to 100 % of B in 30 min, $t_R$ = 17 min; $^1H$ NMR (400 MHz, $CD_3OD$) δ 7.38 - 7.25 (m, 5H), 5.08 (s, 2H), 3.99 (q, *J* = 5.8 Hz, 1 H), 3.16 (qd, *J* = 13.9, 6.1 Hz, 2H), 2.65 (dd, *J* = 7.3, 5.6 Hz, 1 H), 2.16 (s, 3H), 1.10 (d, *J* = 7.0 Hz, 3H). $^{13}C$ NMR (101 MHz, $CD_3OD$) δ 213.6, (C=O carbamate and Cipso not detected), 129.4, 129.0, 128.9, 71.6, 67.5, 51.2, 45.8, 29.0, 11.0. **Minor regioisomer (*S*)-*N*-Cbz-6-amino-5-hydroxyhexan-3-one (25).** The product was purified by preparative HPLC and eluted with a step gradient of $H_2O$:$CH_3CN$ from 100:0 to 50:50, flow rate 1 mL $min^{-1}$ and detection at 215 nm, yielding 12 mg of a 68:32 mixture of **23** + **25.** $[\alpha]_D^{20}$= + 0.15 (c = 0.8 in MeOH); HPLC:10 to 100% of B in 30 min, $t_R$ = 18 min; $^1H$ NMR (400 MHz, $CD_3OD$) δ 7.45 - 7.09 (m, 5H), 5.05 (s, 2H), 4.09 (dt, *J* = 11.6, 5.9 Hz, 1 H), 3.17 - 3.10 (m, 2H), 2.54 - 2.50 (m, 2H), 2.46 (q, *J* = 7.3 Hz, 2H), 0.98 (t, *J* = 7.3 Hz, 3H). $^{13}C$ NMR (101 MHz, $CD_3OD$) δ (C=O carbamate and carbonyl and Cipso not detected), 128.0, 127.6, 127.4, 66.6, 66.1, 46.3, 46.0, 36.0, 6.4.

The intramolecular reductive amination reaction of **23+24** produced four diastereoisomers in 80:8:8:4 rate. Major (80%). **(3*S*,4*R*,5*R*)-4,5-dimethylpyrrolidin-3-ol (26):** $^1H$ NMR (500 MHz, $CD_3OD$) δ 3.99 (td, *J* = 6.6, 4.9 Hz, 1H), 3.40 (dd, *J* = 12.1, 6.9 Hz, 1H), 3.14 (dq, *J* = 9.7, 6.6 Hz, 1 H), 3.06 - 2.99 (m, 1 H), 1.79 - 1.69 (m, 1 H), 1.39 (d, *J* = 6.6 Hz, 3H), 1.11 (d, *J* = 6.9 Hz, 3H). $^{13}C$ NMR (101 MHz, $CD_3OD$) δ 75.2, 60.4, 50.1, 47.5, 15.3, 13.3. Minor (8%) **(3*S*,4*R*,5*S*)-4,5-dimethylpyrrolidin-3-ol (27):** $^1H$ NMR (500 MHz, $CD_3OD$) δ 4.15 (dt, *J* = 4.5, 2.0 Hz, 1 H), 3.98 - 3.90 (m, 1 H), 3.53 - 3.46 (m, 1 H), 3.11 - 3.06 (m, 1 H), 2.26 - 2.19 (m, 1 H), 1.31 (d, *J* = 7.0 Hz, 3H), 0.92 (d, *J* = 7.5 Hz, 3H). $^{13}C$ NMR (101 MHz, $CD_3OD$) δ 75.6, 56.7, 50.5, 44.1, 11.6, 9.9. Minor (8%) **(3*R*,4*R*,5*R*)-4,5-dimethylpyrrolidin-3-ol (28):** $^1H$ NMR (500 MHz, $CD_3OD$) δ 4.24 (t, *J* = 4.1 Hz, 1 H), 3.43 - 3.40 (m, 1 H), 3.38 - 3.33 (m, 1 H), 3.02 - 2.97 (m, 1 H), 1.88 - 1.77 (m, 1 H), 1.35 (d, *J* = 7.1 Hz, 3H), 1.05 (d, *J* = 2.6 Hz, 3H). $^{13}C$ NMR (101 MHz, $CD_3OD$) δ 71.8, 58.6, 52.1, 45.2, 13.7, 8.8. Minor (4%) **(3*R*,4*R*,5*S*)-4,5-dimethylpyrrolidin-3-ol (29):** $^1H$ NMR (500 MHz, $CD_3OD$) δ 4.30 (td, *J* = 4.7, 2.6 Hz, 1 H), 3.84 - 3.75 (m, 1 H), 3.35 - 3.27 (m, 1 H), 3.19 - 3.14 (m, 1 H), 2.35 - 2.30 (m, 1 H), 1.33 (d, *J* = 10.8 Hz, 3H), 1.05 (d, *J* = 2.6 Hz, 3H). $^{13}C$ NMR (101 MHz, $CD_3OD$) δ 71.8, 57.2, 51.5, 40.0, 13.8, 7.1. ESI-TOF: calculated for $[M+H]^+$ $C_6H_{14}NO$: 116.1075, found 116.1078.

**Example 4.5.**

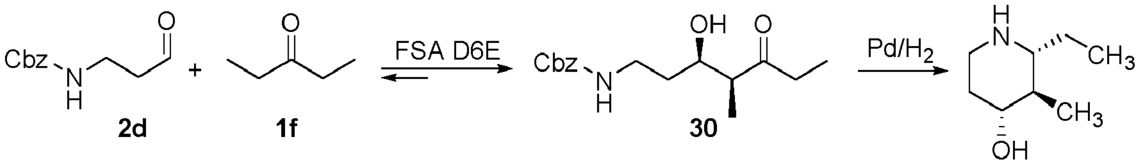


2d 1f 30 31

**(4*S*,5*R*)-*N*-Cbz-7-amino-5-hydroxy-4-methylheptan-3-one (30):** This compound was obtained following the procedure described above. *N*-Cbz-3-aminopropanal **(2d)** (1.6 mmol), 3-pentanone and FSA D6E were used. The product was purified by silica gel column chromatography and eluted with a step gradient of hexane:AcOEt from 1:0 to 3:7, yielding 164.1 mg (35%) of **30.** $[\alpha]_D^{20}$= + 36.5 (c = 2.3 in MeOH); HPLC: 10 to 100% of B in 30 min, $t_R$ = 20 min; $^1$H NMR (400 MHz, $CD_3OD$) δ 7.45 - 7.17 (m, 5H), 5.05 (s, 2H), 3.83 (ddd, *J* = 9.4, 5.6, 3.7 Hz, 1 H), 3.20 (hept, *J* = 7.2 Hz, 2H), 2.62 (p, *J* = 6.8 Hz, 1 H), 2.52 (qd, *J* = 7.2, 2.0 Hz, 2H), 1.67 - 1.39 (m, 2H), 1.08 (d, *J* = 7.0 Hz, 3H), 0.99 (t, *J* = 7.2 Hz, 3H). $^{13}$C NMR (101 MHz, $CD_3OD$) δ 215.2, 157.6, 137.0, 128.0, 127.5, 127.3, 69.4, 65.9, 51.5, 37.5, 34.7, 34.5, 10.4, 6.5.

The intramolecular reductive amination reaction produced **(2*R*,3*R*,4*R*)-2-ethyl-3-methylpiperidin-4-ol (31).** $^1$H NMR (400 MHz, $CD_3OD$) δ 3.27 - 3.25 (m, 2H), 2.87 (td, *J* = 13.1, 2.9 Hz, 1 H), 2.56 (ddd, *J* = 10.6, 7.4, 3.3 Hz, 1 H), 2.02 (ddt, *J* = 13.3, 5.1, 2.8 Hz, 1 H), 1.85 (dqd, *J* = 15.2, 7.6, 2.8 Hz, 1 H), 1.66 - 1.49 (m, 2H), 1.38 (tq, *J* = 10.2, 6.5 Hz, 1 H), 1.05 (d, *J* = 6.5 Hz, 3H), 1.00 (t, *J* = 7.6 Hz, 3H). $^{13}$C NMR (101 MHz, $CD_3OD$) δ 73.1, 62.9, 44.5, 43.3, 34.0, 25.3, 13.8, 9.3. ESI-TOF: calculated for $[M+H]^+$ $C_8H_{18}NO$: 144.1388, found 144.1385

**Example 4.6.**

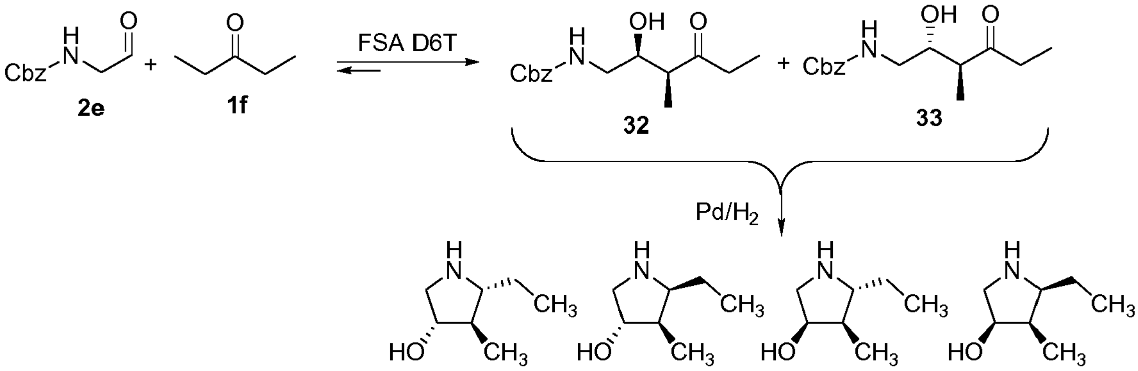


2e 1f 32 33 34 35 36 37

**(4*S*,5*S*)-*N*-Cbz-6-amino-5-hydroxy-4-methyl-hexan-3-one (32) and (4*S*,5*R*)-*N*-Cbz-6-amino-5-hydroxy-4-methyl-hexan-3-one (33).** These compounds were obtained following the procedure described above. N-Cbz-glycinal **(2e)** (1.6 mmol), 3-pentanone and FSA D6T were used. The product was purified by silica gel column chromatography and eluted with a step gradient of hexane:AcOEt from 1:0 to 3:7, yielding 134.4 mg (30 %) of **32+33.** $[\alpha]_D^{20}$ = + 12.3 (*c* = 1.26 in MeOH); HPLC:10 to 100 % of B in 30 min, $t_R$= 19 min; $^1$H NMR (400 MHz, $CD_3OD$) δ 7.44 - 7.16 (m, 5H), 5.19 - 5.02 (m, 2H), 3.92 (q, *J* = 5.6 Hz, 1 H), 3.16 - 3.06 (m, 2H), 2.64 (dt, *J* = 12.9, 6.8 Hz, 1 H), 2.51 (q, *J* = 7.2 Hz, 2H), 1.08 (d, *J* = 7.0 Hz, 3H), 0.97 (t, *J* = 7.2 Hz, 3H). $^{13}$C NMR (101 MHz, $CD_3OD$) δ 214.6, 157.6, 136.9, 128.0, 127.5, 127.4, 70.5, 66.1, 49.0, 44.4, 34.3, 10.3, 6.5. Compound **33** was not clearly assigned in the NMR, but it was inferred from the structures observed in the reductive amination products.

The intramolecular reductive amination reaction produced four diastereoisomers in ≈ 79:8:5:8 rate. Major (79): **(*3S,4R,5R*)-5-ethyl-4-methylpyrrolidin-3-ol (34).** $^1$H NMR (400 MHz, $CD_3OD$) δ 3.97 (q, *J* = 5.3 Hz, 1 H), 3.36 - 3.30 (m, 1 H), 3.02 (dd, *J* = 12.0, 4.5 Hz, 1 H), 2.91 (tdd, *J* = 8.7, 5.1, 1.5 Hz, 1H), 1.91 - 1.75 (m, 2H), 1.72 - 1.61 (m, 1H), 1.10 (d, *J* = 7.0 Hz, 3H), 1.03 (t, *J* = 7.5 Hz, 3H). $^{13}$C NMR (101 MHz, $CD_3OD$) δ 75.7, 66.7, 50.3, 46.3, 25.1, 14.5, 9.9. Minor (8): **(*3S,4R,5S*)-5-ethyl-4-methylpyrrolidin-3-ol** (**35**). $^1$H NMR (400 MHz, $CD_3OD$) $^1$H NMR (400 MHz, $CD_3OD$) δ 4.24 (t, *J* = 4.1 Hz, 1 H), 3.41 - 3.34 (m, 1 H), 3.17 - 3.12 (m, 2H), 1.89 - 1.82 (m, 2H), 1.54 - 1.43 (m, 1 H), 1.10 - 1.09 (m, 3H), 1.02 - 0.98 (m, 3H). $^{13}$C NMR (101 MHz, $CD_3OD$) δ 72.0, 64.8, 52.0, 43.6, 23.6, 15.5, 10.1. Minor (5): **(*3R,4R,5S*)-5-ethyl-4-methylpyrrolidin-3-ol (36):** $^1$H NMR (400 MHz, $CD_3OD$) δ 4.14 (d, *J* = 4.5 Hz, 1 H), 3.73 - 3.66 (m, 3H), 3.49 - 3.44 (m, 2H), 3.11 - 3.07 (m, 1 H), 2.32 - 2.24 (m, 1 H), 1.71 - 1.65 (m, 2H), 1.04 (d, *J* = 7.5 Hz, 3H), 0.88 (d, *J* = 7.5

Hz, 3H). $^{13}C$ NMR (101 MHz, $CD_3OD$) δ 75.7, 62.7, 50.6, 43.0, 22.1, 9.9, 9.5. Minor (8): **(*3R,4R,5R*)-5-ethyl-4-methyl-pyrrolidin-3-ol (37):** $^1H$ NMR (400 MHz, $CD_3OD$) δ 4.35 (q, *J* = 5.7 Hz, 1 H), 3.47 - 3.43 (m, 1 H), 3.27 - 3.23 (m, 1 H), 3.08 - 3.04 (m, 1 H), 2.38 (td, *J* = 7.4, 5.0 Hz, 1 H), 1.74 - 1.66 (m, 1 H), 1.06 (d, *J* = 5.1 Hz, 19H), 0.97 (d, *J* = 5.2 Hz, 2H). $^{13}C$ NMR (101 MHz, $CD_3OD$) δ 71.3, 63.1, 49.7, 39.2, 25.9, 9.9, 6.7. ESI-TOF: calculated for $[M+H]^+$ $C_7H_{16}NO$: 130.1231, found 130.1234

**Example 4.7.**

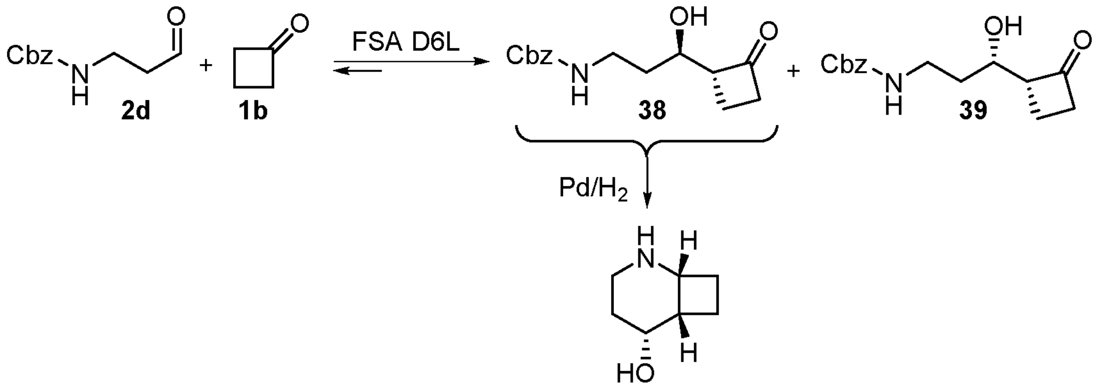


**(*R*)-2-((*R*)-*N*-Cbz-3-amino-1-hydroxypropyl)cyclobutan-1-one (38) and (*R*)-2-((*S*)-*N*-Cbz-3-amino-1-hydroxypropyl)cyclobutan-1-one (39):** These compounds were obtained following the procedure described above in a 80:20 ratio. *N*-Cbz-3-aminopropanal **(2d)** (1.6 mmol), cyclobutanone and FSA D6L were used. The product was purified by silica gel column chromatography and eluted with a step gradient of hexane:AcOEt from 1:0 to 3:7, yielding 201.8 mg (46 %) of product. $[\alpha]_D^{20}$= + 36.49 (*c* = 1.82 in MeOH); HPLC:10 to 100 % of B in 30 min, $t_R$ = 17 min. Major (80%) **(38):** $^1H$ NMR (400 MHz, $CD_3OD$) δ 7.41 - 7.25 (m, 5H), 5.06 (s, 2H), 3.73 (dt, *J* = 9.0, 4.4 Hz, 1 H), 3.47 - 3.35 (m, 1 H), 3.23 (t, *J* = 6.9 Hz, 2H), 3.03 - 2.78 (m, 2H), 2.09 (qd, *J* = 10.3, 5.9 Hz, 1 H), 1.96 (td, *J* = 10.6, 5.2 Hz, 1 H), 1.86 - 1.63 (m, 2H). $^{13}C$ NMR (101 MHz, $CD_3OD$) δ 212.3, 159.0, 138.4, 129.4, 129.0, 128.8, 69.1, 67.4, 66.9, 45.7, 38.7, 36.1, 14.4. Minor (20) **(39):** $^1H$ NMR (400 MHz, $CD_3OD$) δ 7.41 - 7.25 (m, 5H), 5.06 (s, 2H), 3.94 - 3.87 (m, 1 H), 3.21 - 3.10 (m, 1 H), 3.22 - 3.15 (m, 2H), 3.01 - 2.80 (m, 2H), 2.14 - 1.90 (m, 2H), 1.71 - 1.52 (m, 2H). $^{13}C$ NMR (101 MHz, $CD_3OD$) δ 212.3, 159.0, 138.4, 129.4, 129.0, 128.8, 67.2, 67.1, 66.8, 45.9, 38.5, 36.5, 13.5. The intramolecular reductive amination reaction produced **(1*R*,2*R*,6*R*)-1,2,6-trimethylbicyclo[4.2.0]octane (40).** $^1H$ NMR (400 MHz, $CD_3OD$) δ 3.77 (dt, *J* = 10.7, 5.7 Hz, 1 H), 3.63 - 3.55 (m, 1 H), 3.17 (dt, *J* = 13.2, 4.2 Hz, 1 H), 2.74 (t, *J* = 7.1 Hz, 1 H), 2.62 (ddd, *J* = 13.7, 12.0, 2.9 Hz, 1 H), 2.34 - 2.19 (m, 2H), 1.96 - 1.78 (m, 2H), 1.78 - 1.63 (m, 2H). $^{13}C$ NMR (101 MHz, $CD_3OD$) δ 64.2, 52.9, 40.3, 37.3, 28.1, 24.4, 18.4. ESI-TOF: calculated for $[M+H]^+$ $C_7H_{14}NO$: 128.1075, found 128.1070

**Example 4.8.**

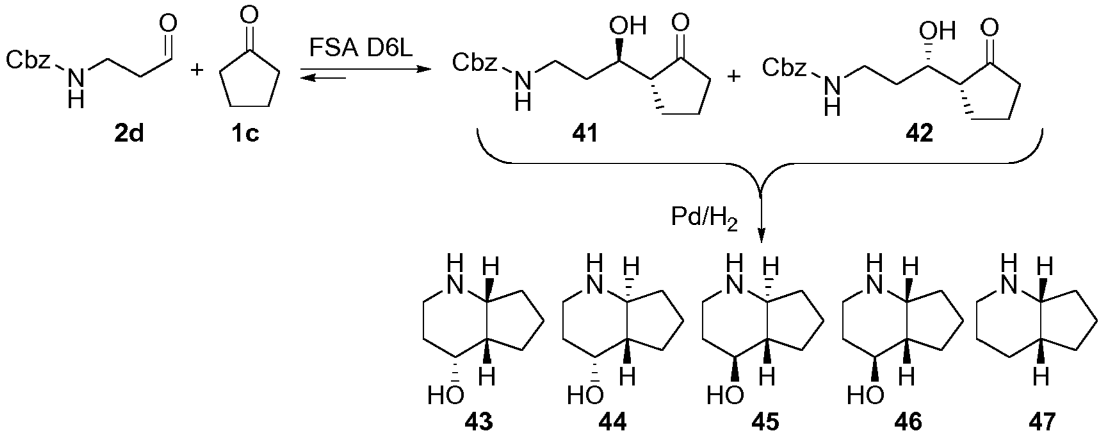


2d 1c 41 42

43 44 45 46 47

**(*R*)-2-((*R*)-*N*-Cbz-3-amino-1-hydroxypropyl)cyclopentan-1-one (41) and (*R*)-2-((*S*)-*N*-Cbz-3-amino-1-hydroxypropyl)cyclopentan-1-one (42).** These compounds were obtained following the procedure described above in a 67:33 ratio. *N*-Cbz-3-aminopropanal **(2d)** (1.6 mmol), cyclopentanone **(1c)** and FSA D6L were used. The product was purified by silica gel column chromatography and eluted with a step gradient of hexane:AcOEt from 1:0 to 3:7, yielding 303.5 mg (65%) of product. HPLC:10 to 100% of B in 30 min, $t_R$ = 18 and 19 min. Major (67%) **(41):** $^1$H NMR (400 MHz, $CD_3OD$) δ 7.41 - 7.17 (m, 5H), 5.06 (s, 2H), 3.78 (ddd, *J* = 9.1, 5.4, 3.4 Hz, 1 H), 3.27 - 3.12 (m, 2H), 2.35 - 2.21 (m, 2H), 2.18 - 2.09 (m, 3H), 1.83 - 1.71 (m, 3H), 1.70 - 1.61 (m, 1 H). $^{13}$C NMR (101 MHz, $CD_3OD$) δ 221.4, 157.5, 137.0, 128.0, 127.5, 127.3, 69.1, 66.1, 53.7, 38.7, 37.4, 25.8, 22.6, 20.2. Minor (33%) **(42):** $^1$H NMR (400 MHz, $CD_3OD$) δ 7.41 - 7.17 (m, 5H), 5.06 (s, 2H), 4.06 (ddd, *J* = 8.4, 4.7, 3.2 Hz, 1 H), 3.28 - 3.16 (m, 2H), 2.28 - 2.18 (m, 1 H), 2.18 - 2.09 (m, 5H), 1.83 - 1.71 (m, 1 H), 1.70 - 1.61 (m, 2H). $^{13}$C NMR (101 MHz, $CD_3OD$) δ 220.9, 157.5, 137.0, 128.0, 127.5, 127.3, 66.1, 66.8, 55.2, 38.9, 38.5, 36.3, 24.0, 21.5.

The intramolecular reductive amination reaction produced four diastereoisomers including a dehydroxylated analogue **47** in a 15:11:15:22:37 ratio. Selected signals of the NMR spectra. Minor (15%): **(4*R*,4a*S*,7a*R*)-octahydro-1*H*-cyclopenta[*b*]pyridin-4-ol (43).** $^1$H NMR (500 MHz, MeOD) δ 4.19 (dt, *J* = 10.6, 5.2 Hz, 1 H), 3.64 (m, 1 H), 3.36 (m, 1 H), 3.02 (m, 1H), 2.49 (m, 1 H), 2.19 - 1.59 (m, 6H). $^{13}$C NMR (101 MHz, $CD_3OD$) δ 65.5, 59.0, 44.6, 42.3. Minor (11%): **(4*R*,4a*S*,7a*S*)-octahydro-1*H*-cyclopenta[*b*]pyridin-4-ol (44).** $^1$H NMR (500 MHz, MeOD) δ 3.99 (td, *J* = 4.7, 3.1 Hz, 1 H), 3.77 (m, 1 H), 3.32 (m, 1 H), 3.17 (dt, *J* = 12.8, 4.4 Hz, 1 H), 2.22 (m, 1 H), 2.19 - 1.59 (m, 7H). $^{13}$C NMR (101 MHz, $CD_3OD$) δ 63.2, 55.4, 45.4, 37.9. Minor (15%): **(4*S*,4a*S*,7a*S*)-octahydro-1*H*-cyclopenta[*b*]pyridin-4-ol (45).** $^1$H NMR (500 MHz, MeOD) δ 3.62 (m, 1 H), 3.50 (m, 1 H), 3.08 (dd, *J* = 13.6, 3.5 Hz, 1 H), 2.90 (td, *J* = 11.3, 7.1 Hz, 1 H), 2.17 (m, 1 H), 2.19 - 1.59 (m, 8H). $^{13}$C NMR (101 MHz, $CD_3OD$) δ 70.5, 59.9, 49.7, 45.0. Minor (22%): **(4*S*,4a*S*,7a*R*)-octahydro-1*H*-cyclopenta[*b*]pyridin-4-ol (46).** $^1$H NMR (500 MHz, MeOD) δ 3.48 (m, 1 H), 3.47 (m, 1H), 3.01 (m, 1 H), 2.78 (td, *J* = 11.2, 7.1 Hz, 2H), 2.14 (m, 1 H), 2.19 - 1.59 (m, 8H). $^{13}$C NMR (101 MHz, $CD_3OD$) δ 61.4, 57.9, 43.9, 42.1. Major (37%) dehydroxylated analogue: **(4a*R*,7a*R*)-octahydro-1*H*-cyclopenta[*b*]pyridine (47):** $^1$H NMR (500 MHz, MeOD) δ 3.58 (ddd, *J* = 7.9, 5.4, 2.9 Hz, 4H), 3.27 (t, *J* = 3.7 Hz, 2H), 3.01 (m, 1 H), 2.35 - 2.25 (m, 1 H), 2.11 (m, 1H), 1.79 (m, 2H), 1.85 (m, 2H), 1.71 (m, 1H), 1.76 (m, 2H). $^{13}$C NMR (101 MHz, $CD_3OD$) δ 57.8, 42.3, 37.1, 28.2, 26.2, 22.4, 20.5, 17.8. ESI-TOF: calculated for $[M+H]^+$ $C_8H_{16}NO$: 142.1231, found 142.1238

**Example 4.9.**

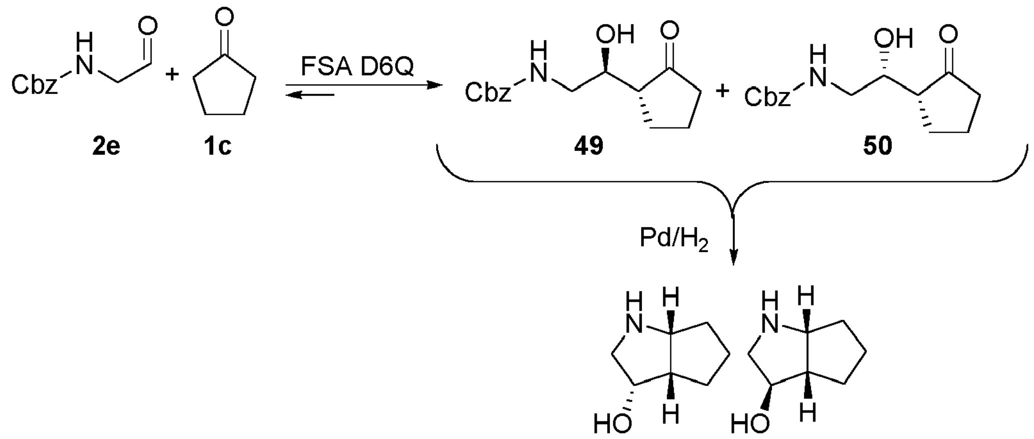


2e 1c 49 50 51 52

**(*R*)-2-((*S*)-*N*-Cbz-2-amino-1-hydroxyethyl)cyclopentan-1-one (49) and (*R*)-2-((*R*)-*N*-Cbz-2-amino-1-hydroxyethyl)cyclopentan-1-one (50).** These compounds were obtained following the procedure described above in a 50:50 ratio; HPLC: 10 to 100 % of B in 30 min, $t_R$ = 17 and 18 min, respectively. *N*-Cbz-glycinal **(x)** (1.6 mmol), cyclopentanone and FSA D6Q were used. The product was purified by silica gel column chromatography and eluted with a step gradient of hexane:AcOEt from 1:0 to 3:7, yielding two fraction pools: fraction pool 1 containing **49** pure (53 mg,) and fraction pool 2 as a 1:2 mixture of **49:50** (117.2 mg), total 170.2 mg (38 %) of product. **(*R*)-2-((*S*)-*N*-Cbz-2-amino-1-hydroxyethyl)cyclopentan-1-one (49).** $^1$H NMR (400 MHz, $CD_3OD$) δ 7.38 - 7.24 (m, 5H), 5.07 (s, 2H), 4.09 (td, *J* = 6.9, 3.0 Hz, 1H), 3.23 (dd, *J* = 13.7, 6.2 Hz, 1H), 3.14 (dd, *J* = 13.7, 7.2 Hz, 1 H), 2.33 - 2.15 (m, 2H), 2.15 - 1.92 (m, 4H), 1.83 - 1.70 (m, 1 H). $^{13}$C NMR (101 MHz, $CD_3OD$) δ 222.2, 159.0, 138.4, 129.4, 129.0, 128.9, 69.6, 67.5, 53.2, 46.3, 39.8, 23.7, 21.7. **Fraction pool 2 49:50** 33:67 ratio. NMR: **(*R*)-2-((*R*)-*N*-Cbz-2-amino-1-hydroxyethyl)cyclopentan-1-one (50):** $^1$H NMR (400 MHz, $CD_3OD$) δ 7.42 - 7.16 (m, 5H), 5.07 (s, 2H), 3.83 (q, *J* = 5.5 Hz, 1 H), 3.38 - 3.29 (m, 2H), 2.32 - 1.98 (m, 5H), 1.91 - 1.71 (m, 2H). $^{13}$C NMR (101 MHz, $CD_3OD$) δ 222.5, 138.5, 129.4, 128.8, 70.8, 67.5, 52.6, 45.7, 40.1, 27.5, 21.8.

The intramolecular reductive amination reaction of the fraction pool 2 (**49:50** 33:67 ratio) produced two diastereoisomers the corresponding to the aldol adducts **51** and **52,** respectively. From aldol adduct **49: (3*S*,3a*S*,6a*R*)-octahydrocyclopenta[*b*]pyrrol-3-ol (51).** $^1$H NMR (500 MHz, $CD_3OD$) δ 4.35 (dt, *J* = 6.7, 3.5 Hz, 1 H), 3.99 (ddd, *J* = 9.0, 7.2, 3.3 Hz, 1 H), 3.15 (d, *J* = 3.5 Hz, 2H), 2.81 (tdd, *J* = 9.3, 6.4, 3.6 Hz, 1 H), 2.00 - 1.92 (m, 1 H), 1.93 - 1.86 (m, 2H), 1.89 - 1.75 (m, 2H), 1.67 - 1.62 (m, 1 H). $^{13}$C NMR (101 MHz, $CD_3OD$) δ 70.6, 64.6, 53.9, 47.1, 30.8, 26.0, 24.9. From aldol adduct **50**: **(3*R*,3a*S*,6a*R*)-octahydrocyclopenta[*b*]pyrrol-3-ol (52).** $^1$H NMR (500 MHz, $CD_3OD$) δ 4.19 - 4.12 (m, 2H), 3.20 - 3.15 (m, 1 H), 3.09 (dt, *J* = 12.1, 1.6 Hz, 1 H), 2.65 (ddd, *J* = 9.6, 7.9, 6.3 Hz, 1 H), 1.99 - 1.92 (m, 1 H), 1.93 - 1.87 (m, 1 H), 1.69 - 1.67 (m, 1 H), 1.68 - 1.61 (m, 1 H), 1.57 - 1.51 (m, 1 H), 1.40 (dq, *J* = 13.3, 6.7 Hz, 1 H). $^{13}$C NMR (101 MHz, $CD_3OD$) δ 75.6, 63.0, 51.7, 51.6, 31.7, 29.1, 24.9. ESI-TOF: calculated for $[M+H]^+$ $C_7H_{14}NO$: 128.1075, found 128.1079.

### Example 5. Scale up of aldol addition of ethanal

#### Example 5.1.

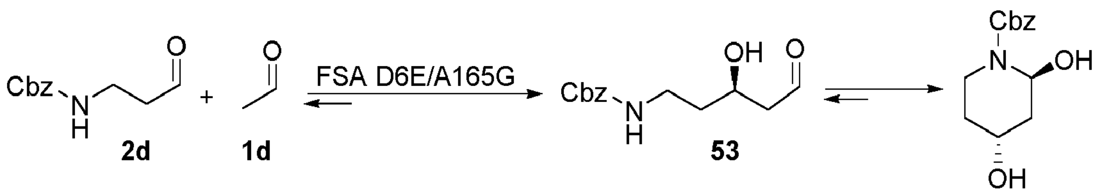


2d 1d 53 54

**(2*S*,4*R*)-*N*-Cbz-piperidine-2,4-diol (54):** The reaction was conducted in a 50 mL screw capped conical-bottom polypropylene tubes. To a FSA D6E/A165G (3 mg $mL^{-1}$) solution in plain water (18.9 mL), 1 M triethanolamine buffer, pH 8 (1 mL) was added. To this solution, *N*-Cbz-3-aminopropanal (331.2 mg, 80 mM) and acetaldehyde (100 mM, 112 μL) were added. Total reaction volume was 20 mL. Reaction mixtures were shaken (1000 rpm) at 25 °C for 24 h. After

that, the reaction was stopped with MeOH (30 mL) to precipitate the enzyme, and the mixture was filtered through Celite. MeOH was evaporated and the reaction was lyophilized. The product was purified by silica gel column chromatography and eluted with a step gradient of hexane:AcOEt from 1:0 to 3:7, yielding 191.3 mg (47%) of the title compound. $[\alpha]_D^{20}$= + 8.51 (*c* = 2.1 in MeOH); HPLC: 10 to 100% of B in 30 min, $t_R$ = 14.5 min; $^1$H NMR (400 MHz, $CD_3OD$) δ 7.59 - 7.24 (m, 5H), 5.58 - 5.47 (m, 1 H), 5.22 - 4.99 (m, 2H), 4.11 - 4.06 (m, 1 H), 4.03 (tt, *J* = 11.3, 4.5 Hz, 1 H), 3.12 - 2.98 (m, 1 H), 2.24 - 2.13 (m, 1 H), 2.02 - 1.92 (m, 1 H), 1.94 - 1.84 (m, 1 H), 1.49 - 1.42 (m, 1 H), 1.42 - 1.33 (m, 1 H). $^{13}$C NMR (101 MHz, $CD_3OD$) δ 155.7, 136.5, 128.2, 127.8, 127.5, 83.0, 67.1, 63.4, 38.7, 37.0, 35.1, 31.2. Rotamer: $^1$H NMR (400 MHz, $CD_3OD$) δ 7.59 - 7.24 (m, 5H), 5.49 - 5.43 (m, 1H), 5.16 - 5.08 (m, 2H), 4.03 - 3.99 (m, 1 H), 3.97 - 3.91 (m, 1 H), 3.45 - 3.38 (m, 1 H), 2.12 - 2.04 (m, 1 H), 1.86 (dt, *J* = 14.7, 3.9 Hz, 1 H), 1.81 - 1.70 (m, 2H). $^{13}$C NMR (101 MHz, $CD_3OD$) δ 155.4, 136.5, 128.2, 127.8, 127.5, 82.5, 67.1, 60.0, 35.2, 33.0, 31.3. ESI-TOF: calculated for $[M+H]^+$ $C_{13}H_{18}NO_4$: 252.1235, found 252.1231

**Example 6. Aldol addition propanal to formaldehyde.**

**Example 6.1.**

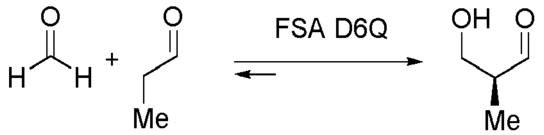


55 56 57

Reaction was conducted in 250 mL Erlenmeyer. To a FSA D6Q (3 mg $mL^{-1}$, 300 mg) solution in plain water (93 mL), 1 M triethanolamine buffer pH 8 (5 mL) was added. To this solution, formaldehyde (10 mmol, 813 μL of a 12.3 M solution) and propanal (10 mmol, 725 μL) were added. Total reaction volume was 99.5 mL. Reaction mixtures were shaken (200 rpm) at 25 °C for 48 h. *Coupling reaction to structurally characterize the aldol adduct **57.***

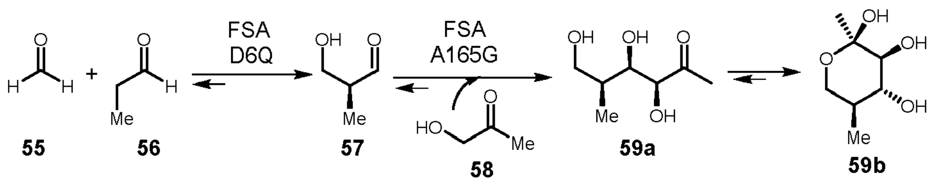


55 56 57 58 59a 59b

After 48 hours hydroxyacetone (**58**) (10 mmol, 680 μL) and FSA A165G (3 mg $mL^{-1}$, 300 mg) were added to the reaction and shaken (200 rpm) at 25 °C during an additional 24 hours. After that, the reaction was stopped with MeOH (100 mL) to precipitate the enzyme, and the mixture was filtered through activated charcoal. MeOH was evaporated and the reaction was lyophilized. The product was purified by silica gel column chromatography eluted with step gradient $CHCl_3$:MeOH from 1:0 to 95:5, yielding 392.2 mg (25%) of product (**59b**). Product **59b** was structurally characterized by NMR. *Enzymatic Aldehyde oxidation to acid. Formation of 2-hydroxyisobutyrate (12).*

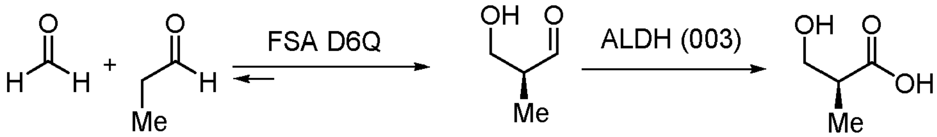


55 56 57 60

Aldehyde dehydrogenase from Prozomix (ALDH (003), KIT: Aldehyde dehydrogenase Panel, Cat. No.: PRO-ALDH (003) LOT: 2016-1) was used. To the reaction mixture containing **57** (12 mL, ≈100 mM of **11**), $NAD^+$ (226.36 μL from a 66.3 mM stock solution, final concentration 1 mM), and NOX (PROZO) (1254.48 μL from a 119.6 mg $mL^{-1}$ stock solution) were added (final concentration of **57** = 80 mM, total volume 15 mL). The reaction was started by adding AIDH (771.7 μL from a 194.4 mg $mL^{-1}$ stock solution) and the reaction placed in a shaker at 1000 rpm at room temperature. After 3 hours, the conversion of aldol adduct to **60** was approximately 96.4%.

HPLC analysis of propanal, formaldehyde and aldol adduct: Samples (5 μL) were withdrawn immediately after

adding the enzyme and after 24 hours, and *O*-benzylhydroxylamine (50 μL, of a 0.13 mM stock solution in pyridine:methanol:water 33:15:2) and placed at room temperature for 5 min. After that, methanol (450 μL) was added and subsequently injected (30 μL) to the HPLC system. HPLC analysis: column: XBridge® C18, 5 μm, 4.6 x 250 mm column (Waters), solvent system A: 0.1 % (v/v) TFA in $H_2O$; solvent B: 0.095% (v/v) TFA in $CH_3CN$:$H_2O$ 80:20, gradient elution from 10 to 100 % of B over 30 min, 30 °C, flow rate 1 mL $min^{-1}$ and detection 215 nm.

*2-Hydroxyisobutyrate (***60***) identification by reaction with 2,4'-dibromo-acetophenone*: After the reaction was finished, MeOH was added (150 mL; 10xreaction volume) and the enzymes precipitated were filtered off using Celite. Methanol was then evaporated and the residue was lyophilized. The solid obtained was dissolved in DMF (10 mL) and (1.5 mmol) was added. After 7 hours of reaction, the mixture was diluted in ethyl acetate (50 mL), silica gel (50 g) was then added and solvent was evaporated to dryness. Product purification was carried by column chromatography (47x4.5 cm) on silica gel (100 g) with a gradient elution: hexane/ethyl acetate 100:0 (200 mL), 90:10 (200 mL); 80:20 (200 mL), 70:30 (200 mL) 60:40 (200 mL) and 50:50 (1 L), product eluted at 50:50. Fractions (10 mL) were analyzed by TLC. Fractions containing pure product was pooled, the solvent evaporated obtaining 102.3 mg of 4-bromophenacyl-β-hydroxyisobutyrate. Product was characterized by NMR analysis. HPLC Analysis was done on RP-HPLC Xbridge C18 (5 μm, 4.6 x 250 mm, Waters) column. Solvent system: A trifluoroacetic acid (TFA) (0.1% v/v) in water, solvent B (0.095% TFA) in $CH_3CH$:water 4:1; Gradient elution 10-100% B, 30 min, flow-rate 1 mL $min^{-1}$, 30°C and detection at 215 nm. The retention time of 4-bromophenacyl-β-hydroxyisobutyrate was 21 min.

**Example 7. Aldol addition of ethanal to 2-chloroethanal.**

**Example 7.1.**

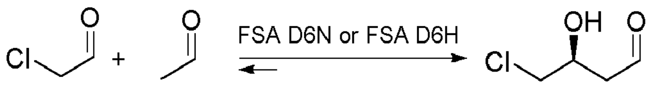


61 **1d** 62

The volume of the reaction was 300 μL but formally it can be scaled up to 5-50 mL (or other amount if needed). 2-Chloroethanal (100 mM) and acetaldehyde (100 mM) were dissolved in 50 mM triethanolamine buffer pH 7.5 (the amount depending on the reaction volume). The reaction was started by the addition of FSA D6N (3 mg $mL^{-1}$). The reaction mixture was shacked (1000 rpm) at 25°C. After 24 h another extra addition of FSA D6N (3 mg $mL^{-1}$) can be done if the reaction is not complete (max conversion around 80-85%). When the reaction is completed MeOH (1 x reaction volume) is added. The resulting mixture was filtered over activated charcoal. The filtrate was evaporated under vacuum, dissolved in MeOH and adsorbed onto silica. The product adsorbed onto silica was loaded on a column chromatography (3 cm diameter) packed with silica (15 cm of silica bed), sample was eluted with hexane:ethyl acetate mixture in a step gradient manner: 100:0 (100 mL), 70:30 (200 mL), 50:50 (400 mL) and 30:70 (400 mL). The product appeared around the 50:50 hexane:ethyl acetate mixture. Product was compared with an authentic sample obtained using DERA catalyst.

HPLC analysis: Samples (5 μL) were withdrawn immediately after adding the enzyme and after 24 hours, and O-benzylhydroxylamine (50 μL, of a 0.13 mM stock solution in pyridine:methanol:water 33:15:2) was added and placed at room temperature for 5 min. After that, methanol (450 μL) was added and subsequently injected (30 μL) to the HPLC system (column: XBridge® C18, 5 μm, 4.6 x 250 mm column (Waters)). HPLC analysis: solvent A: 0.1 % TFA in $H_2O$; solvent B: 0.095% TFA in $CH_3CN$:$H_2O$ 80:20, gradient elution from 10 to 100 % of B over 30 min.

**Example 8. Aldol addition of propanone to propanal.**

**Example 8.1.**

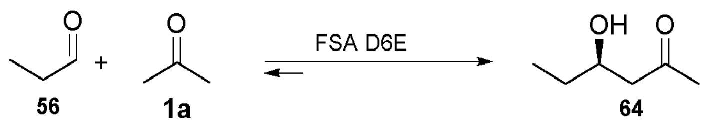


56 **1a** 64

**(R)-4-hydroxyhexan-2-one (64):** In the reaction vessel lyophilized whole cells of *E. coli* BL21, previously

cultivated for plasmid-instructed expression of FSA D6E (250 mg, equivalent to 15 mg of pure FSA) were suspended in PBS buffer (15 mL, pH 7.0). Acetone (4 mL, 54.5 mmol), freshly distilled propanal (**56**) (1 mL, 13.9 mmol), and DTT (2 mM) were added. The suspension was shaken on a rotary shaker at 21 °C for 4 days, then centrifuged at 3087xg for 20 min. The supernatant was extracted with diethyl ether (3 x 20 mL), and the combined organic layers were dried over $MgSO_4$, filtered, and the solvent evaporated under vacuum. The oily residue containing almost pure product **64** (550 mg, 33.9%) was analyzed by NMR spectroscopy. $^1H$ NMR (300 MHz, MeOD): $\delta$ = 1.05 (t, 3H, *J* (6/5) = 7.5 Hz, H-6), 1.58 (m, 2H, H-5), 2.29 (s, 3H, H-1), 2.68 (d, 2H, *J* (3/4) = 6.4 Hz, H-3), 4.06 (tt, 1 H, H-4). $^{13}C$ NMR (75 MHz, MeOD): $\delta$ = 10.24 (C-6), 30.80 (C-1), 30.93 (C-5), 51.23 (C-3), 70.05 (C-4), 210.80 (C-2).

**Example 9. Aldol addition of propanone to *n*-butanal**

**Example 9.1**

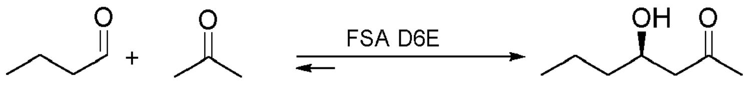


**65** **1a** **66**

(**R**)-**4**-**hydroxyheptan**-**2**-**one (66):** In the reaction vessel, lyophilized whole cells of *E. coli* BL21, previously cultivated for plasmid-instructed expression of FSA D6E (200 mg, equivalent to 12 mg of pure FSA) were suspended in PBS buffer (15 mL, pH 7.0). Acetone **(1a)** (4 mL, 54.5 mmol), freshly distilled butanal **(65)** (1 mL, 11.1 mmol), and DTT (2 mM) were added. The suspension was shaken on a rotary shaker at 21 °C for 4 days, then centrifuged at 3087×*g* for 20 min. The supernatant was extracted with diethyl ether (3 x 20 mL), and the combined organic layers were dried over $MgSO_4$, filtered, and the solvent evaporated under vacuum. The oily residue containing almost pure product **66** (141 mg, 9.7%) was analyzed by NMR spectroscopy. $^1H$ NMR (300 MHz, MeOD): $\delta$ = 0.99 (t, 3H, *J* (7/6) = 7.1 Hz, H-7), 1.47 (m, 4H, H-5,6), 2.22 (s, 3H, H-1), 2.61 (d, 2H, *J* (3/4) = 6.1 Hz, H-3), 4.08 (tt, 1 H, H-4). $^{13}C$ NMR (75 MHz, MeOD): $\delta$ = 14.33 (C-7), 19.75 (C-6), 30.71 (C-1), 40.60 (C-5), 51.86 (C-3), 68.60 (C-4), 210.85 (C-2).

**Example 10. Aldol addition of propanone to *n*-pentanal**

**Example 10.1**

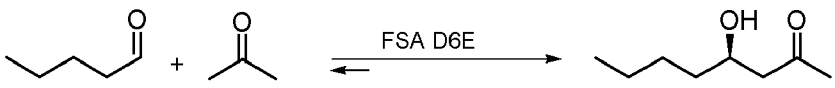


**67** **1a** **68**

**(*R*)-4-hydroxyoctan-2-one (68):** In the reaction vessel, lyophilized whole cells of *E. coli* BL21, previously cultivated for plasmid-instructed expression of FSA D6E (250 mg, equivalent to 15 mg of pure FSA) were suspended in PBS buffer (15 mL, pH 7.0). Acetone **(1a)** (4 mL, 54.5 mmol) and freshly distilled pentanal **(67)** (1 mL, 9.4 mmol) were added. The suspension was shaken on a rotary shaker at 21 °C for 6 days, then centrifuged at 3087xg for 20 min. The supernatant was extracted with diethyl ether (3 x 20 mL), and the combined organic layers were dried over $MgSO_4$, filtered, and the solvent evaporated under vacuum. The oily residue containing almost pure product **68** (250 mg, 18.4%) was analyzed by NMR spectroscopy. $^1H$ NMR (300 MHz, $CDCl_3$): $\delta$ = 0.81 (t, 3H, *J* = 6.4 Hz, H-8), 1.15-1.48 (m, 6H, H-5, 6, 7), 2.09 (s, 3H, H-1), 2.50 (m, 2H, H-3), 3.95 (tt, 1 H, *J* = 7.4/3.4 Hz, H-4). $^{13}C$ NMR (75 MHz, $CDCl_3$ ): $\delta$ = 13.95 (C-8), 22.56 (C-7), 27.60 (C-6), 30.69 (C-1), 36.20 (C-5), 50.08 (C-3), 67.57 (C-4), 209.89 (C-2).

**Example 11. Aldol addition of propanone to isopentanal**

**Example 11.1**

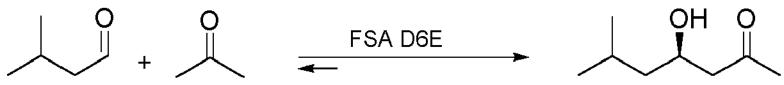


69 1a 70

**(R)-4-hydroxy-6-methylheptan-2-one (70):** In the reaction vessel lyophilized whole cells of *E. coli* BL21, previously cultivated for plasmid-instructed expression of FSA D6E (191 mg, equivalent to 11 mg of pure FSA) were suspended in PBS buffer (15 mL, pH 7.0). Acetone **(1a)** (4 mL, 54.5 mmol), freshly distilled isopentanal **(69)** (1 mL, 9.3 mmol), and DTT (2 mM) were added. The suspension was shaken on a rotary shaker at 21 °C for 4 days, then centrifuged at 3087×*g* for 20 min. The supernatant was extracted with diethyl ether (3 x 20 mL), and the combined organic layers were dried over $MgSO_4$, filtered, and the solvent evaporated under vacuum. The oily residue containing almost pure product **70** (284 mg, 21.2%) was analyzed by NMR spectroscopy. $^1H$ NMR (300 MHz, $CDCl_3$): $\delta$ = 0.87 (d, 6H, *J* = 6.6 Hz, H-7/1'), 1.09 (m, 1 H, *J* = 13.7 Hz, H-6), 1.42 (m, 1 H, *J* = 13.7 Hz, H-5a), 1.74 (m, 1 H, H-5b), 2.13 (s, 3H, H-1), 2.52 (m, 2H, H-3), 4.08 (m, 1 H, *J* = 4.4 Hz, H-4). $^{13}C$ NMR (75 MHz, $CDCl_3$): $\delta$ = 22.06 (C-6), 23.35 (C-7/1'), 24.39 (C-7/1'), 30.84 (C-1), 45.58 (C-5), 50.57 (C-3), 65.69 (C-4), 210.15 (C-2).

**Example 12. Aldol addition of propanal to propanal**

**Example 12.1**

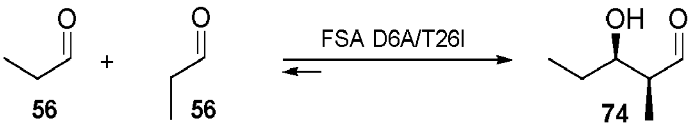


56 56 74

**(2S,3R)-3-hydroxy-2-methylpentanal (74).** The enzyme catalyst FSA D6A/T261 (35 mg) was dissolved in triethanolamine buffer (100 mL; 50 mM, pH 7.4) containing DTT (2 mM). After addition of freshly distilled propanal (1500 μL, 20.9 mmol) the vessel was stoppered and reaction mixture stirred at 20 °C for 3 days. Then $CaCl_2$ solution was added (1 mM final concentration) followed by proteinase K (51 U), and the pH adjusted to 7.0 using satd aq $NaHCO_3$ solution. This mixture was incubated at 55 °C for 2 h during which the turbid solution became clear. The product was extracted with ethyl acetate (4 x 60 mL) with TLC control. The combined organic layers were dried ($MgSO_4$), filtered, and the solvent evaporated at 40 °C under vacuum. The oily residue was purified by silica column chromatography using cyclohexane-ethyl acetate (3:1) as eluent to give pure product (230 mg, 9.5%). $^1H$ NMR (300 MHz, $CDCl_3$): $\delta$ = 0,79 (d, 3H, J=7,05 Hz, H-5), 0,84 (t ,3H, J= 7,45 Hz, H-2'), 1,35 (m ,2H, H-4), 1,65 (m ,1H, H-2), 3,50 (m ,2H, H-3), 3,58 (m ,1H, H-1), 3,96 (brs, 2H, OH-1', OH-3'). $^{13}C$ NMR (75 MHz, $CDCl_3$): $\delta$ = 9,90 (C-5), 10,57 (C-2'), 26,71 (C-4), 38,75 (C-2), 66,19 (C-1), 74,96 (C-3).

*Reduction of aldehyde **74** and diol protection for configurational assignment.*

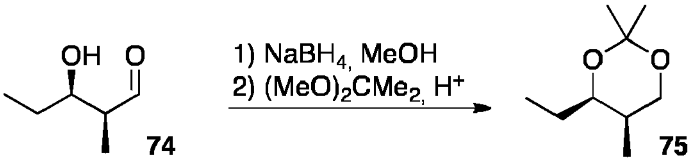


74 75

**(4R,5R)-4-Ethyl-2,2,5-trimethyl-1,3-dioxane (75).** The aldehyde **74** (1 eq) was dissolved in MeOH (1.4 mL per mmol aldehyde) and treated portion wise with $NaBH_4$ (1.5 eq per mmol) with stirring at room temperature. After 2 h, 2 volumes of brine was added and a solid precipitated. The mixture was slowly treated with deionisized water until the turbidity disappeared (ca. 4 mL/mL MeOH). The solution was extracted with ethyl acetate (6x20 mL; TLC control). The combined organic phases were dried ($MgSO_4$), filtered and concentrated under vacuum at 40 °C. The residue (130 mg, 55.5%) was used directly in the next step without further purification.

The diol was taken up in dry acetone (2.5 mL) followed by addition of dimethoxypropane (2.5 mL). To this was

added molecular sieves (250 mg; 3 A) and a catalytic quantity of p-TosOH. The vessel was closed by using a septum and stirred at 20 °C for 2.5 h. After addition of satd $NaHCO_3$ solution (2.5 mL) the mixture was extracted using diethyl ether (3 x 5 mL). The combined organic phases were dried ($MgSO_4$), filtered and concentrated under vacuum to furnish the acetal **75** (300 mg, 29.9 %). $^1H$ NMR (300 MHz, $CDCl_3$, 30 % $C_6D_6$): δ = 1,54 (t ,3H, *J* (4',3') = 7,4 Hz, H-4'), 1,73 (d, 3H, *J* (5',5) = 6,8 Hz, H-5'), 1,83 (m, 1 H, H-5), 1,95 (ddq, 1 H, *J* (3',4') = 7,4 Hz, H-3'a), 2,03 (s, 3H, H-1'), 2,14 (s, 3H, H-2'), 2,20 (ddt, 1 H, *J* (3',4') = 7,4 Hz, H-3'b), 4,16 (dd, *J* (6b,5) = 1,66 , *J* (6a,6b) = 11,40 Hz, 1 H, H-6a), 4,32 (ddd, 1 H, *J* = 2,5 ,5,9 ,8,1 Hz, H-4), 4.56 (dd, *J* (6a,5) = 2,8 , *J* (6b,6a) = 11,40 Hz, 1 H, H-6b) ppm. $^{13}C$ NMR (75 MHz, $CDCl_3$ 30 % $C_6D_6$): δ = 9,56 (C-5'), 10,33 (C-4'), 18,87 (C-1'), 29,81 (C-3'), 29,80 (C-2'), 31,31 (C-5), 66,65 (C-6), 72,82 (C-4), 98,22 (C-2) ppm.

**Example 13. Aldol addition of butanal to butanal**

**Example 13.1**

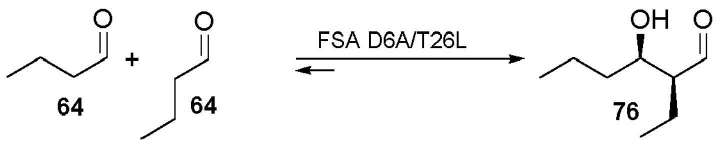


**(2S,3R)-2-ethyl-3-hydroxyhexanal (76):** The enzyme catalyst FSA D6A/T26L (35 mg) was dissolved in triethanolamine buffer (100 mL; 50 mM, pH 7.4) containing DTT (2 mM). After addition of freshly distilled butanal **(64)** (400 μL, 20.9 mmol) the vessel was stoppered and reaction mixture stirred at 20 °C for 3 days. Then $CaCl_2$ solution was added (1 mM final concentration) followed by proteinase K (51 U), and the pH adjusted to 7.0 using satd aq $NaHCO_3$ solution. This mixture was incubated at 55 °C for 2 h during which the turbid solution became clear. The product was extracted using ethyl acetate (4 x 60 mL) with TLC control. The combined organic layers were dried ($MgSO_4$), filtered, and the solvent evaporated at 40 °C under vacuum. The oily residue was purified by silica column chromatography using cyclohexane-ethyl acetate (3:1) as eluent to give pure product **76** (90 mg, 26.9%). $^1H$ NMR (300 MHz, $CHCl_3$): $\delta$ = 1,31 (t, 6H, J = 7,5 Hz, H-5', H-7'), 1,36 (m, 1 H, J = 10,5 Hz, H-5), 1,66 (m, 2H, H-4a', H-6a'), 1,72-1,85 (m, 2H, H-4b', H-6b'), 1,77 (s, 3H, H-1'), 1,78 (s, 3H, H-2'), 1,90 (m, 1 H, H-3a'), 2,12 (m, 1 H, J=3,3 Hz, H-3b'), 4,17 (dd, 1 H, J (6a,5) = 1,8 J (6a,6b) = 11,8 Hz, H-6a), 4,24 (m, 2H, H-4, H-6b) ppm. $^{13}C$ NMR (75 MHz, $CHCl_3$): $\delta$ = 12,06 (C-7'), 14,06 (C-5'),16,15 (C-4'), 18,83 (C-6'), 19,15 (C-1'), 29,79 (C-2'), 34,88 (C-3'), 39,11 (C-5), 62,63 (C-6), 71,94 (C-4), 98,44 (C-2) ppm.

*Reduction of aldehyde **76** and diol protection for configurational assignment.*

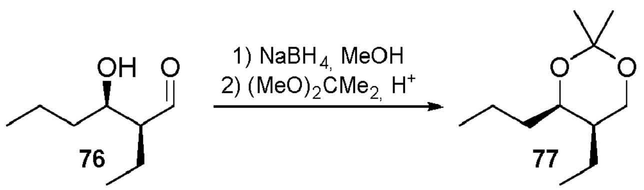


**(4R,5R)-5-Ethyl-2,2-dimethyl-4-propyl-1,3-dioxane (77).** The aldehyde **76** (1 eq) was dissolved in MeOH (1.4 mL per mmol aldehyde) and treated portion wise with $NaBH_4$ (1.5 eq per mmol) with stirring at room temperature. Work-up and acetal protection using dimethoxypropane in acetone with acid catalysis was essentially performed as for **75.** Extraction of product **77** was performed using cyclohexane, and purification was achieved by silica gel column chromatography (ethyl acetate-cyclohexane 1:10). $^1H$ NMR (300 MHz, $CDCl_3$, 30 % $C_6D_6$): δ = 1,54 (t, 3H, *J* (4',3') = 7,4 Hz, H-4'), 1,73 (d, 3H, *J* (5',5) = 6,8 Hz, H-5'), 1,83 (m, 1 H, H-5), 1,95 (ddq, 1 H, *J* (3',4') = 7,4 Hz, H-3'a), 2,03 (s, 3H, H-1'), 2,14 (s, 3H, H-2'), 2,20 (ddt, 1 H, *J* (3',4') = 7,4 Hz, H-3'b), 4,16 (dd, *J* (6b,5) = 1,66 , *J* (6a,6b) = 11,40 Hz, 1 H, H-6a), 4,32 (ddd, 1 H, *J* = 2,5 ,5,9 ,8,1 Hz, H-4), 4.56 (dd, *J* (6a,5) = 2,8 , *J* (6b,6a) = 11,40 Hz, 1H, H-6b) ppm. $^{13}C$ NMR (300 MHz, $CDCl_3$ 30 % $C_6D_6$): $\delta$ = 9,56 (C-5'), 10,33 (C-4'), 18,87 (C-1'), 29,81 (C-3'), 29,80 (C-2'), 31,31 (C-5), 66,65 (C-6), 72,82 (C-4), 98,22 (C-2) ppm.

**Claims**

1. A fructose-6-phosphate aldolase variant comprising:

    a. an amino acid sequence having at least 80%, preferably at least 90%, more preferably at least 99%, sequence identity with SEQ ID NO: 1, and
    b. an amino acid substitution at position 6, corresponding to positions 1 to 220 of SEQ ID NO: 1.

2. The variant according to claim 1, wherein the amino acid substitution is by an amino acid selected from the list consisting of: A, L, N, Q, S, T, E, H, V, G, I or P, preferably by H, N, Q, L, T, E or A.

3. The variant according to any of claims 1 or 2, wherein said variant further comprises an amino acid substitution at position 26, corresponding to positions 1 to 220 of SEQ ID NO: 1.

4. The variant according to claim 3, wherein the amino acid substitution at position 26 is by an amino acid selected from the list consisting of: V, A, L, I or P, preferably by L or I.

5. The variant according to any of claims 1 or 2, wherein said variant further comprises an amino acid substitution at position 165, corresponding to positions 1 to 220 of SEQ ID NO: 1, by the amino acid G.

6. The variant according to any one of claims 1 to 5, wherein said variant comprises the amino acid substitution D6N or D6H.

7. A nucleic acid sequence encoding the variant according to any one of claims 1 to 6.

8. A gene construct comprising the nucleic acid sequence according to claim 7.

**9.** A host cell comprising the nucleic acid sequence according to claim 7 or the gene construct according to claim 8.

**10.** Use of the host cell according to claim 9 for producing the variant according to any one of claims 1 to 6.

**11.** Use of the variant according to any one of claims 1 to 6 or the host cell according to claim 9 as a biocatalyst for aldol carboligation reactions.

**12.** Use according to claim 11, wherein the aldol carboligation reaction takes place between two aldehydes or between an aldehyde and a ketone, preferably wherein the aldehydes are selected from the list consisting of: 2-chloroethanal, formaldehyde, benzyloxyethanal, propanal, pentanal, isopentanal, ethanal, butanal, 3-hydroxypropanal, 3-hydroxybutanal, N-Cbz-3-aminopropanal or N-Cbz-glycinal, and the ketone is selected from the list consisting of: propanone, butanone, cyclobutanone, cyclopentanone or 3-pentanone.

**13.** A method for aldol carboligation comprising the following steps:

a. reacting two aldehydes or an aldehyde and a ketone in the presence of the variant according to any one of claims 1 to 6 or the host cell according to claim 9, and
b. recovering the product of the reaction performed in step (a).

**14.** The method according to claim 13, wherein the aldehydes are selected from the list consisting of: 2-chloroethanal, formaldehyde, benzyloxyethanal, propanal, pentanal, isopentanal, ethanal, butanal, 3-hydroxypropanal, 3-hydroxybutanal, N-Cbz-3-aminopropanal or N-Cbz-glycinal, and the ketone is selected from the list consisting of: propanone, butanone, cyclobutanone, cyclopentanone or 3-pentanone.

Europäisches Patentamt
European Patent Office
Office européen des brevets

# EUROPEAN SEARCH REPORT

Application Number
EP 17 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | Huan Ma: "Aldolases for Enzymatic Carboligation . Diected Evolution and Enzyme Structure-Function Relationschip Studies", Digital Comprehensive Summaries of Uppsala Dissertations from the Faculty of Science and Technology , 2015, pages 11-67, XP002771128, Acta Universitatis Upsaliensis, Uppsala ISBN: 978-91-554-9411-7 Retrieved from the Internet: URL:https://uu.diva-portal.org/smash/get/diva2:869186/FULLTEXT01.pdf [retrieved on 2017-06-15] * abstract * * page 41 - page 52 * ----- | 1-14 | INV. C12N9/88 C12P7/26 C12P7/38 C12P7/24 |
| A | SCHURMANN M ET AL: "Fructose-6-phosphate aldolase is a novel class I aldolase from Escherichia coli and is related to a novel group of bacterial transaldolases.", THE JOURNAL OF BIOLOGICAL CHEMISTRY, vol. 276, no. 14, 6 April 2001 (2001-04-06), pages 11055-11061, XP002771129, ISSN: 0021-9258 * abstract * ----- | 1-14 | TECHNICAL FIELDS SEARCHED (IPC): C12N C12P |

-/--

The present search report has been drawn up for all claims

| Place of search | Date of completion of the search | Examiner |
|---|---|---|
| The Hague | 15 June 2017 | Gurdjian, Didier |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SUDAR MARTINA ET AL: "Mathematical model for aldol addition catalyzed by twod-fructose-6-phosphate aldolases variants overexpressed inE. coli", JOURNAL OF BIOTECHNOLOGY, vol. 167, no. 3, 2013, pages 191-200, XP028703518, ISSN: 0168-1656, DOI: 10.1016/J.JBIOTEC.2013.07.008 * abstract * ----- | 1-14 | |
| A,D | SZEKRENYI ANNA ET AL: "Engineering the donor selectivity of D-fructose-6-phosphate aldolase for biocatalytic asymmetric cross-aldol additions of glycolaldehyde.", CHEMISTRY (WEINHEIM AN DER BERGSTRASSE, GERMANY), vol. 20, no. 39, 22 September 2014 (2014-09-22), pages 12572-12583, XP002771130, ISSN: 1521-3765 * abstract; table 1 * ----- | 1-14 | TECHNICAL FIELDS SEARCHED (IPC) |
| A | GUTIERREZ MARIANA ET AL: "Supplementary Information to Structure-guided redesign of D-fructose-6-phosphate aldolase from E. coli: remarkable activity and selectivity towards acceptor substrates by two-point mutation.", CHEMICAL COMMUNICATIONS, vol. 47, no. 20, 28 May 2011 (2011-05-28), pages S1-S29, XP002771132, (CAMBRIDGE, ENGLAND) ISSN: 1364-548X * page s11 - page s12 * ----- | 1-14 | |

-/--

The present search report has been drawn up for all claims

2

| Place of search | Date of completion of the search | Examiner |
|---|---|---|
| The Hague | 15 June 2017 | Gurdjian, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SZEKRENYI ANNA ET AL: "Asymmetric assembly of aldose carbohydrates from formaldehyde and glycolaldehyde by tandem biocatalytic aldol reactions.", NATURE CHEMISTRY, vol. 7, no. 9, September 2015 (2015-09), pages 724-729, XP002771131, ISSN: 1755-4349 * abstract; table 1 * ----- | 1-14 | |
| A | ZHONG-YU YOU ET AL: "Characterization and application of a newly synthesized 2-deoxyribose-5-phosphate aldolase", JOURNAL OF INDUSTRIAL MICROBIOLOGY & BIOTECHNOLOGY, vol. 40, no. 1, 22 November 2012 (2012-11-22), pages 29-39, XP035156349, OFFICIAL JOURNAL OF THE SOCIETY FOR INDUSTRIAL MICROBIOLOGY, SPRINGER, BERLIN, DE ISSN: 1476-5535, DOI: 10.1007/S10295-012-1213-Y * abstract * ----- | 1-14 | TECHNICAL FIELDS SEARCHED (IPC) |
| A | WO 2008/025826 A1 (CONSEJO SUPERIOR INVESTIGACION [ES]; BIOGLANE S L N E [ES]; CLAPES SAB) 6 March 2008 (2008-03-06) * abstract; claims 1-9; figure 1; sequence 2 * ----- | 1-14 | |

The present search report has been drawn up for all claims

2

| Place of search | Date of completion of the search | Examiner |
|---|---|---|
| The Hague | 15 June 2017 | Gurdjian, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 17 38 2194

This annex lists the patent family members relating to the patent documents cited in the above-mentioned European search report. The members are as contained in the European Patent Office EDP file on
The European Patent Office is in no way liable for these particulars which are merely given for the purpose of information.

15-06-2017

| Patent document cited in search report | Publication date | Patent family member(s) | Publication date |
|---|---|---|---|
| WO 2008025826 A1 | 06-03-2008 | AT 457036 T | 15-02-2010 |
| | | AU 2007291195 A1 | 06-03-2008 |
| | | CA 2661772 A1 | 06-03-2008 |
| | | CN 101522905 A | 02-09-2009 |
| | | DK 2061892 T3 | 25-05-2010 |
| | | EP 2061892 A1 | 27-05-2009 |
| | | ES 2298057 A1 | 01-05-2008 |
| | | ES 2340967 T3 | 11-06-2010 |
| | | JP 5145338 B2 | 13-02-2013 |
| | | JP 2010501198 A | 21-01-2010 |
| | | US 2010009417 A1 | 14-01-2010 |
| | | WO 2008025826 A1 | 06-03-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 103145540
- US 8742176 B
- EP 1734129 A

### Non-patent literature cited in the description


- **RUCIGAJ ; KRAJNC.** *Chem. Eng. J.,* 2015, vol. 259, 11-24
- **ANNA SZEKRENYI et al.** *Chemistry A European Journal,* 2014
- **GUTIERREZ et al.** *Chem. Commun.,* 2011, vol. 47 (20), 5762-5764
- **HUAN MA.** *Digital Comprehensive Summaries of Uppsala Dissertations from the Faculty of Science and Technology,* 2015, vol. 1318, 67
- **HIGGINS.** *CABIOS,* 1989, vol. 5, 151-153
- **WILBUR Y LIPMAN.** *Proceedings of the National Academy of Science USA,* 1983, vol. 80, 726-730
- **DEVEREUX et al.** Nucleic Acids Research. Genetics Computer Group University of Wisconsin, 1984, vol. 12, 287
- **ALTSCHUL et al.** *J. Mol. Biol.,* 1999, vol. 215, 403-410